(19) Europäisches Patentamt
European Patent Office
Office européen des brevets

(11) Numéro de publication: **0 316 218 B1**

(12) **FASCICULE DE BREVET EUROPEEN**

(45) Date de publication de fascicule du brevet: **15.09.93**

(51) Int. Cl.[5]: **C07K 5/08**, A61K 37/02

(21) Numéro de dépôt: **88402761.6**

(22) Date de dépôt: **03.11.88**

Le dossier contient des informations techniques présentées postérieurement au dépôt de la demande et ne figurant pas dans le présent fascicule.

(54) **Nouveaux dérivés de l-proline, leur préparation et leurs applications biologiques.**

(30) Priorité: **03.11.87 FR 8715228**

(43) Date de publication de la demande:
**17.05.89 Bulletin 89/20**

(45) Mention de la délivrance du brevet:
**15.09.93 Bulletin 93/37**

(84) Etats contractants désignés:
**AT BE CH DE ES FR GB GR IT LI LU NL SE**

(56) Documents cités:

**CHEMICAL ABSTRACTS, vol. 97, 1982, page 270, résumé no. 195014r, Columbus, Ohio, US; L. SACHS et al.: "A highly specific aminotripeptidase of rat brain cytosol. Substrate specificity and effects of inhibitors", & BIOCHIM. BIOPHYS. ACT. 1982, 706(2), 229-38**

**CHEMICAL ABSTRACTS, vol. 80, 1974, page 233, résumé no. 93707a, Columbus, Ohio, US; H. SHIRAISHI et al.: "Taste of proline-containing peptides", & AGR. BIOL. CHEM. 1973, 37(10), 2427-8**

(73) Titulaire: **INORGAN SA**
**Recherche & Développement pharmaceutiques Case Postale 162 World Trade Center**
**CH-1215 Genève 15(CH)**

(72) Inventeur: **Fiez-Vandal, Pierre-Yves**
**Ouai de l'Amiral-Mouchez 2**
**F-78400 Chatou(FR)**

(74) Mandataire: **Peaucelle, Chantal et al**
**Cabinet Armengaud Aine 3, avenue Bugeaud**
**F-75116 Paris (FR)**

**EP 0 316 218 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention a pour objet de nouveaux dérivés de la L-proline, leur préparation et leurs applications biologiques.

L'invention vise plus particulièrement des dérivés de la L-proline ayant une activité thérapeutique, en particulier une activité psychotrope et plus spécialement nootrope.

Divers dérivés de L-proline ont déjà été décrits mais pour des activités différentes. Ainsi, dans la demande de brevet EP 0173510, on décrit des dérivés de tripeptides renfermant deux motifs proline en indiquant que ces produits sont dotés d'une activité antihypertensive.

Dans le Chemical Abstracts vol. 105, 1986, 4622b, on décrit un dérivé de L-proline, à savoir le [$^3$H] benzoyl phénylalanyl-alanyl-proline, pour rapporter son utilisation comme substrat de l'enzyme de conversion de l'angiotensine.

Quant aux produits à activité nootrope connus, ils comprennent l'ergot de seigle, le piracétam et certaines neurohormones peptidiques. L'inventeur de la présente demande de brevet a observé que ces produits présentent une analogie de structure et a cherché à élaborer des dérivés de L-proline présentant en particulier un effet nootrope, mais ne possédant pas les activités pharmacologiques de l'ergot liées à l'ergoline, plus actifs que le piracétam et actifs par voie orale.

Les travaux effectués ont permis de développer une famille de dérivés de L-proline présentant les propriétés recherchées et, de manière avantageuse, actifs à faible dose.

Les dérivés de L-proline de l'invention sont caractérisés en ce qu'ils répondent à la formule I. dans laquelle

- $R_1$ est un groupe de formule II

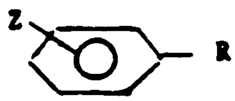

(II)

dans laquelle R est un radical carbonyle CO-, un radical acyle Y-CO- ou un radical oxy-acyle O-Y-CO-, dans lesquels Y est une chaîne alcoyle ou alcényle, notamment de 1 à 4 atomes de carbone, Z représente un ou plusieurs atomes d'hydrogène, ou un ou plusieurs substituants en position ortho et/ou ortho' et/ou méta et/ou meta' et/ou para, choisi(s) parmi les atomes d'halogène, le groupe $CF_3$, les radicaux alcoyle ou alcoxy de 1 à 4 atomes de carbone et pour deux substituants voisins un groupe alcoylènedioxy, le groupe alcoylène renfermant de 1 à 3 atomes de carbone,

- $R_2$ est un radical $NH_2$ ou OH,ou des dérivés fonctionnels de ces radicaux,
- $A_1$ et $A_2$, identiques ou différents l'un de l'autre, sont des résidus d'acides aminés, et
- $B_1$ et $B_2$, identiques ou différents l'un de l'autre, représentent un atome d'hydrogène ou un groupe méthyle, et les sels pharmacologiquement acceptables de ces dérivés.

Dans une famille préférée, le substituant Z dans la formule II représente un atome d'hydrogène.

Dans un autre famille préférée, le substituant Z est un atome d'halogène choisi parmi le chlore ou le fluor, un groupe $CF_3$, un radical alcoxy choisi parmi un groupe méthoxy ou éthoxy, et pour deux positions voisines, un radical alcoylène-dioxy choisi parmi le 3,4-méthylène- ou 3,4-éthylène-dioxy.

Dans ces familles, le substituant R, est avantageusement choisi parmi les groupes CO-, $CH_2$-CO-, $CH_2$-$CH_2$-CO-, $CH_2$-$CH_2$-$CH_2$-CO-, CH = CH-CO- ou O-$CH_2$-CO-.

Un groupe préféré de dérivés de L-proline conforme aux familles ci-dessus comprend des résidus d'acides aminés $A_1$ et $A_2$ naturels. Dans des produits avantageux $A_1$ est choisi parmi les résidus glycine, L-alanine et L-valine, et $A_2$ parmi les résidus de glycine, L-phénylalanine, L-histidine, L-leucine, L-valine et L-alanine.

L'invention vise également un procédé de préparation des dérivés de L-proline définis ci-dessus.

Ce procédé est caractérisé par le fait qu'on fait réagir

- un dérivé de formule III :

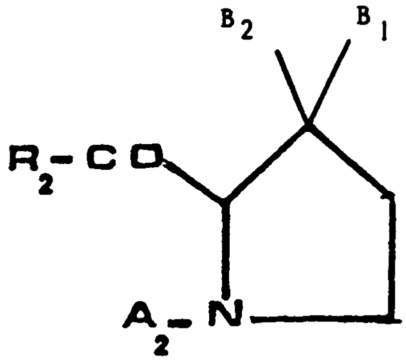

III

- avec un dérivé de formule IV

$R_1$ - $A_1$ (IV)

Dans ces formules, $B_1$, $B_2$, $A_1$, $A_2$, $R_1$ et $R_2$ sont tels que définis ci-dessus.

Pour obtenir le dérivé de formule III, on met avantageusement en oeuvre un dérivé de proline de formule V :

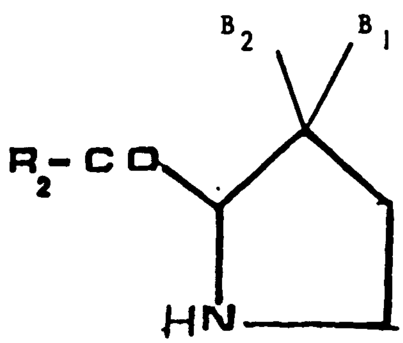

V

et un acide aminé $A_2$.

La réaction de condensation est de préférence réalisée à une température inférieure à l'ambiante, plus particulièrement inférieure à 0 ° C. Des températures préférées sont inférieures à -10 ° C, notamment de l'ordre de - 15 ° C.

On opère avantageusement en milieu solvant organique tel que le tétrahydrofuranne (THF) ou le diméthylformamide (DMF).

La fonction amine de l'acide aminé $A_2$ mis en oeuvre est avantageusement protégée par un groupe éliminable ensuite par des acides. On utilise des groupes protecteurs classiques en synthèse peptidique tel que le groupe tertiobutyloxycarbonyle, benzyloxycarbonyle ou 9-fluorénylméthyloxycarbonyle, ou tout autre groupe connu de l'homme de l'art utilisé dans la chimie peptidique.

Le groupe protecteur est éliminé en vue de la condensation de $A_2$ avec le dérivé de proline de formule III, avantageusement dans des conditions acides fortes. On utilise, à cet effet, un acide anhydre tel qu'une solution de chlorure d'hydrogène gazeux dans un solvant organique, par exemple le dioxanne.

Des conditions acides fortes en milieu aqueux peuvent être également utilisées. Parmi ces conditions, on citera l'utilisation d'acide chlorhydrique ou sulfurique en solution aqueuse concentrée.

Le dérivé $R_1$-$A_1$ de formule IV résulte de la condensation de dérivés réactifs de $R_1$ et de $A_1$, (la fonction carboxyle de $A_1$ étant bloquée par un groupe protecteur), suivie d'une saponification à l'aide d'une base forte. Les dérivés réactifs de $R_1$ comprennent par exemple les halogénures, notamment le chlorure, ces groupes réactifs étant utilisables, étant donné qu'il n'y a pas possibilité de réactions secondaires avec les dérivés d'acides aminés protégés. La fonction carboxyle de $A_1$ est bloquée par les groupes utilisés

habituellement en synthèse peptidique. Cette fonction est de préférence bloquée sous forme d'esters notamment d'ester alcoylique, le groupe alcoyle ayant de préférence de 1 à 4 atomes de carbone, ou encore d'ester benzylique. Avantageusement, les esters de $A_1$ sont sous forme de sels, par exemple de chlorhydrates.

L'étape de condensation entre les dérivés réactifs est réalisée de préférence à température inférieure à l'ambiante, de préférence inférieure à 0 °C, notamment de l'ordre de -5 à -10 °C.

L'étape de saponification avec une base est réalisée en milieu solvant organique, notamment dans un solvant alcoolique tel que le méthanol. La réaction est réalisée avantageusement à température ambiante.

Lorsque le produit précipite, le pH est ajusté à des valeurs acides, inférieures à 3, notamment inférieures à 2, de préférence de l'ordre de 1,5.

Les acides aminés $A_1$ et $A_2$ mis en oeuvre dans les réactions de condensation ci-dessus sont avantageusement protégés et activés en vue du couplage. De nombreuses méthodes sont utilisables à cet effet.

Des résultats satisfaisants sont obtenus en utilisant par exemple la N-méthylmorpholine, pour préparer le sel du dérivé d'acide aminé, que l'on fait réagir ensuite avec l'isobutyl chloroformate pour former un anhydride mixte carboxylique-carbonique qui est un dérivé activé de l'acide aminé. Pour éviter toute réaction secondaire résultant de l'utilisation du chloroformate d'isobutyle, on a recours à un additif constitué avantageusement par un dérivé de triazole, notamment le 1-hydroxybenzotriazole.

Parmi les autres méthodes utilisées de manière classique, pour activer des acides aminés en vue d'un couplage, on citera la formation d'esters actifs, tels que ceux dérivés notamment du N-hydroxysuccinimide, du 4-nitrophénol, du pentafluorophénol ou analogue, ainsi que de l'utilisation pour le couplage de carbodiimides, succinimides et analogues.

L' étude pharmacologique des dérivés de L-proline de l'invention a mis en évidence une activité psychotropique élevée.

Cette activité a été révélée en particulier dans les tests pharmacologiques d'évitement passif, communément utilisés pour déterminer l'activité potentielle de produits notamment sur la mémoire.

Ainsi, en induisant une amnésie par absorption de scopolamine, on constate que les produits de l'invention sont capables et ce selon un aspect de grand intérêt, à faible dose, de corriger l'amnésie ainsi induite.

En outre, les propriétés avantageuses des composés de l'invention s'accompagnent d'une grande innocuité.

En effet, les essais réalisés selon le test d'Irwin sur la souris montrent que les produits de l'invention n'entrainent pas de mort, ni de convulsion des animaux jusqu'à des doses de plus de 1000 mg.kg-1.

Ces produits sont donc particulièrement appropriés pour l'élaboration de compositions pharmaceutiques.

Les compositions pharmaceutiques de l'invention renferment une quantité efficace d'au moins un dérivé de L-proline tel que défini ci-dessus, en association avec un véhicule pharmaceutique inerte.

Des compositions pharmaceutiques avantageuses renferment ces dérivés seuls ou en association avec des médicaments psychotropes antidépresseurs, neuroleptiques, ou la L-dopa.

Compte tenu de leur activité nootrope, ces compositions pharmaceutiques sont utilisables notamment dans les indications thérapeutiques suivantes : troubles de la mémoire, démence sénile, maladie d'Alzheimer, maladie de Parkinson, schizophrénie, dépression, neuropathies périphériques et troubles neuromoteurs.

Les compositions pharmaceutiques de l'invention sont administrables sous différentes formes, à savoir par voie injectable, nasale, rectale, orale.

Pour l'administration par voie orale, on a recours en particulier à des comprimés, pilules, tablettes, gélules, gouttes ou encore à des liposomes. Ces compositions renferment avantageusement de 1 à 100 mg par unité de prise, de préférence de 2,5 à 50 mg.

D'autres formes d'administration comprennent des solutions injectables par voie intra-veineuse, sous-cutanée ou intra-musculaire, dans des solutions stériles ou stérilisables.

Ces solutions renferment par unité de prise de 1 à 50 mg, de préférence de 0,5 à 50 mg.

A titre indicatif, la posologie utilisable chez l'homme correspond aux doses suivantes : ainsi on administre par exemple au patient 5 à 300 mg/jour en une ou plusieurs prises.

L'invention vise encore les réactifs biologiques dont les principes actifs sont constitués par les dérivés de L-proline définis plus haut.

Ces réactifs peuvent être utiles comme références ou étalons dans des études d'éventuelles activités nootropes.

D'autres caractéristiques et avantages de l'invention apparaîtront dans les exemples qui suivent relatifs à la préparation de dérivés de L-proline et à l'étude de leur activité nootropique.

EXEMPLE 1 :

Synthèse du cinnamoyl-glycyl-L-phénylalanyl-L-prolineamide de formule VI :

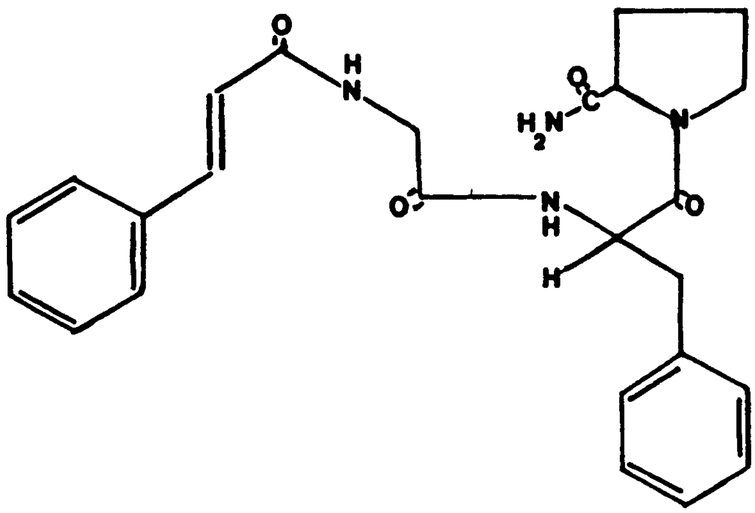

(VI)

Cette synthèse comporte cinq étapes réalisées comme suit :

1/ Préparation du t-Boc-L-phénylalanyl-L-prolineamide

On met en oeuvre les composés suivants selon les quantités indiquées :

| | |
|---|---|
| t-Boc-L-phénylalanine | 40 g |
| tétrahydrofuranne | 200 ml |
| N-méthylmorpholine | 21 ml |
| isobutyl-chloroformate | 19,5 ml |
| 1-hydroxybenzotriazole | 23 g |
| diméthylformamide | 75 ml |
| L-prolineamide | 17,12 g |
| diméthylformamide | 75 ml |

On dissout la t-Boc-L-phénylalanine (tertiobutyloxycarbonyl-L-phénylalamine) dans du tétrahydrofuranne, on refroidit la solution à -15 °C sous agitation magnétique et on la traite avec de la N-méthylmorpholine, puis avec de l'isobutyl-chloroformate. Le mélange réactionnel est soumis à agitation pendant 5 à 10 minutes à basse température, puis on le traite avec une solution dans du diméthylformamide prérefroidie de 1-hydroxybenzotriazole dans du diméthylformamide.

Après agitation pendant 5 à 10 minutes, on ajoute une solution dans du diméthylformamide prérefroidie de prolineamide au mélange réactionnel.

Le mélange est soumis à agitation pendant environ 1 heure à basse température puis on le laisse se réchauffer jusqu'à la température ambiante et on le soumet à agitation durant environ 14 heures.

On dilue le mélange réactionnel avec de l'acétate d'éthyle (800 ml environ), puis on le lave successivement avec une solution aqueuse saturée de chlorure de sodium (à 2 reprises), une solution aqueuse à 5% de bicarbonate de sodium (à 3 reprises), une solution aqueuse saturée de chlorure de sodium (1 fois), une solution aqueuse d'acide chlorydrique 0,5 N (à 3 reprises) et une solution aqueuse saturée de chlorure de sodium (à 3 reprises). On sèche la phase organique sur du sulfate de magnésium, on la filtre puis on évapore sous pression réduite. Le sirop résiduel est trituré avec de l'éther de pétrole pour induire la

cristallisation et le produit obtenu filtré, lavé avec de l'éther de pétrole et séché sous vide.

On obtient 43,7 g de produit. Sa chromatographie en couche mince ($CHCl_3$:MeOH:AcOH, 45:4:1) montre un $R_f$ de 0,5.

2/ Préparation du cinnamoyl-ester méthylique de glycine

| | |
|---|---|
| chlorhydrate de l'ester méthylique de glycine | 28 g |
| N-méthylmorpholine | 39 ml |
| chlorure de cinnamoyle | 33 g |
| tétrahydrofuranne | 250 ml |
| N-méthylmorpholine | 35 ml |

On met en suspension le chlorhydrate de l'ester méthylique de glycine dans du tétrahydrofuranne, on refroidit la solution de -5 à -10° C, puis on la traite avec une première partie aliquote de N-méthylmorpholine. On ajoute immédiatement du chlorure de cinnamoyle et la deuxième partie aliquote de N-méthylmorpholine. Le mélange réactionnel est soumis à agitation durant 30 minutes a basse température puis on le laisse se réchauffer jusqu'à la température ambiante et on le soumet à agitation encore 2 à 3 heures. Après dilution avec de l'acétate d'éthyle (environ 750ml), on lave la solution avec de l'eau (à 3 reprises), une solution aqueuse d'acide chlorhydrique 0,5 N (à 3 reprises) et finalement avec de l'eau. La phase organique est séchée sur du sulfate de magnésium, filtrée et évaporée à sec sous pression réduite. Le résidu est trituré avec de l'éther de pétrole pour induire la cristallisation. On laisse le produit environ 14 heures à 4° C, puis on le filtre et on le sèche sous vide. On l'utilise dans l'étape suivante sans nouvel le purification. On obtient 31 g de produit.

3/ Préparation de la cinnamoyl-glycine :

Les composés suivants, selon les doses indiquées, sont utilisés :

| | |
|---|---|
| cinnamoyl-ester méthylique de glycine | 31 g |
| méthanol | 300 ml |
| hydroxyde de sodium (2 N dans méthanol) | 150 ml |

On dissout l'ester méthylique de glycine et de cinnamoyle dans du méthanol, puis on le traite avec une solution 2 N d'hydroxyde de sodium dans le méthanol. Le mélange réactionnel est soumis à agitation à température ambiante pendant 2 heures, puis dilué avec de l'eau (300 ml). Le pH du mélange est ajusté à 1,5 avec de l'acide chlorhydrique lorsque le produit précipite. On conserve le mélange durant environ 14 heures à 4° C pour compléter la cristallisation, on filtre puis on lave avec de l'eau et on sèche sous vide. Le rendement est de 23 g. Le point de fusion du produit est de 189 à 192°C. La chromatographie en couche mince ($CHCl_3$:MeOH:AcOH, 45:4:1) donne un $R_f$ de 0,2.

4/ Préparation du trifluoacétate de L-phénylalanyl-L-prolineamide :

On utilise les produits suivants :

| | |
|---|---|
| t-Boc-phénylalanyl-L-prolineamide | 30 g |
| dichlorométhane | 300 ml |
| acide trifluoroacétique | 300 ml |

On dissout dans du dichlorométhane le t-Boc-L-phénylalanyl-L-prolineamideet on le traite avec de l'acide trifluoracétique pendant 30 minutes à température ambiante. Le solvant est évaporé sous pression réduite et le résidu réévaporé a partir du toluène. Le résidu huileux est trituré avec de l'éther pour précipiter le produit qui est filtré et séché sous vide. On obtient 33,1 g de produit. La chromatographie sur couche mince dans ($CHCl_3$:MeOH:AcOH, 45:4:1) donne un $R_f$ de 0,3.

5/Préparation du cinnamoyl-glycyl-L-phénylalanyl-L-prolineamide :

La réaction est réalisée en mettant en oeuvre les produits suivants, selon les quantités indiquées.

| | |
|---|---|
| cinnamoyl-glycine | 20 g |
| diméthylformamide | 200 ml |
| 1-hydroxybenzotriazole | 16,3 g |
| dicyclohexylcarbodiimide | 20 g |
| L-phénylalanyl-L-prolineamide.TFA | 33 g |
| diméthylformamide | 200 ml |
| N-méthylmorpholine | 11 ml |

On dissout la cinnamoyl-glycine dans du diméthylformamide, on refroidit à 0° C, puis on ajoute du 1-hydroxybenzotriazole et le mélange est traité avec du dicyclohexylcarbodiimide.

Le mélange réactionnel est soumis à agitation pendant 1 heure à 0° C, puis 1 heure à température ambiante.

Pendant ce temps, on dissout du L-phénylalanyl-L-prolimeamide trifluoroacétate dans du diméthylformamide (200 ml). On refroidit la solution à 0° C et on la traite avec de la N-méthylmorpholine. On ajoute la solution à la solution d'ester de cinnamoyl-glycine active formée précédemment et on soumet le mélange à agitation à température ambiante durant environ 14 heures. Le mélange réactionnel est dilué avec de l'acétate d'éthyle (1,5 litres), filtré, et lavé avec du chlorure de sodium saturé (à 2 reprises), une solution aqueuse de bicarbonate de sodium à 5% (à 3 reprises), une solution de chlorure de sodium saturée (1 fois), de l'acide chlorydrique 0,5 N (à 3 reprises) et finalement du chlorure de sodium saturé (à 3 reprises).

La phase organique est séchée sur du sulfate de magnésium, filtrée et le solvant évaporé sous pression réduite. Le résidu est redissous dans de l'acétone, filtré et le filtrat est évaporé sous pression réduite. Afin d'éliminer la dicyclohexylurée résiduelle, on recommence 2 fois ce processus.Le produit récupéré (13,5 g) est purifié sur du gel de silice (500 g), élué avec du dichlorométhane contenant de 1 à 5% de méthanol. Les fractions contenant le produit pur sont rassemblées et évaporées à sec sous pression réduite. Le résidu est trituré avec de l'éther de pétrole filtré, séché sous vide.

On obtient 11,5 g de produit de $P_F$ 95-115°C. (Cette plage de $P_F$ peut être due à une isomérisation cis-trans). La chromatographie sur couche mince avec ($CHCl_3$:MeOH: AcOH, 45:4:1) donne un $R_f$ de 0,4 et avec (n-BuOH:AcOH:$H_2O$, 4:1:1), un $R_f$ de 0,8. $[\alpha]_D$ = -49.62° (c = 1, MeOH).

Le produit obtenu est insoluble dans l'eau et soluble à raison d'au moins 1 à 5%, dans les solvants organiques tels que le méthanol, le chloroforme, l'acétate d'éthyle ou le diméthylformamide.

EXEMPLE 2 :

Synthèse du 4-fluorocinnamoyl-glycyl-L-phenylalanine-L-prolinamide de formule VII

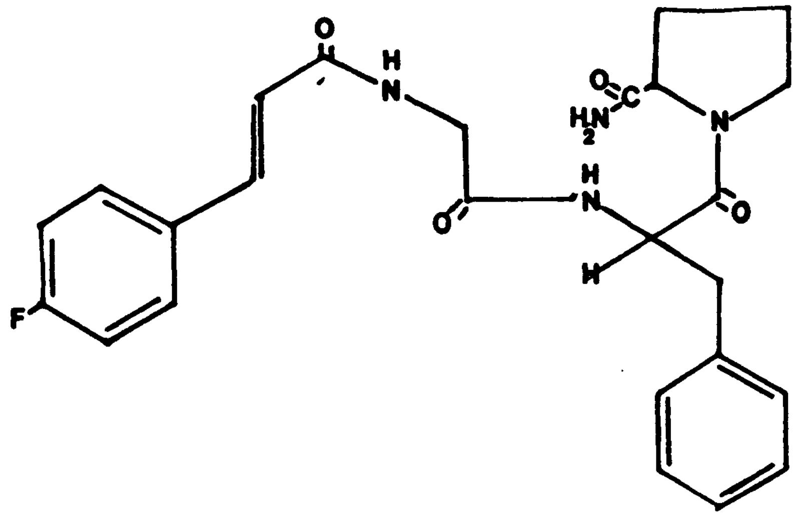

(VII)

1. 4-fluorocinnamoyl-ester méthylique de glycine

En opérant selon le procédé général décrit dans l'exemple 1, étape 2, pour le cinnamoyl-ester méthylique de glycine, on met en suspension le chlorhydrate de l'ester méthylique de glycine (7,5 g) dans du tétrahydrofurane, on refroidit à 0 ° C, on traite avec de la N-méthylmorpholine (8,4 ml, 1 équivalent) puis on ajoute immédiatement une solution pré-refroidie d'acide 4-fluorocinnamique (10,0 g) dans du tétrahydrofurane préactivé par traitement avec du dicyclohexylcarbodiimide (12,4 g) 1 équivalent) et du 1-hydroxybenzotriazole (9,2 g). On obtient le 4-fluorocinnamoyl-ester methylique de glycine sous forme d'un solide blanc utilisé dans l'étape ultérieure de synthèse sans procéder à une nouvelle purification.

| | |
|---|---|
| Rendement | 8,5 g |
| CCM | $CHCl_3$:MeOH:AcOH, 45:4:1): $R_f$ = 0,8 |

2. 4-fluorocinnamoyl-glycine

On soumet 8,5 g du produit résultant de l'étape précédente à une saponification par traitement avec de la soude dans du méthanol comme décrit ci-dessus dans l'exemple 1, étape 3 pour la préparation de la cinnamoyl-glycine.

| | |
|---|---|
| Rendement | 3,4 g |
| CCM | ($CHCl_3$:MeOH:AcOH, 45:4:1): $R_f$ = 0.2 |

3. 4-fluorocinnamoyl-glycyl-L-phénylalanyl-L-prolinamide

On réalise le couplage du produit de l'étape 2 (1,78 g) à du trifluoroacétate de L-phénylalanyl-L-prolinamide (3,0 g) en utilisant du dicyclohexylcarbodiimide en présence de 1-hydroxybenzotriazole. Le produit obtenu se présente sous forme d'un solide blanc.

| | |
|---|---|
| Rendement | 1,4 g |
| CCM | ($CHCl_3$:MeOH:AcOH, 45:4:1): $R_f$ = 0.4 |
| PF | 125 ° C |

EXEMPLE 3 : Synthèse du 3,4-méthylenedioxycinnamoyl-glycyl-L-prolinamide de formule VIII

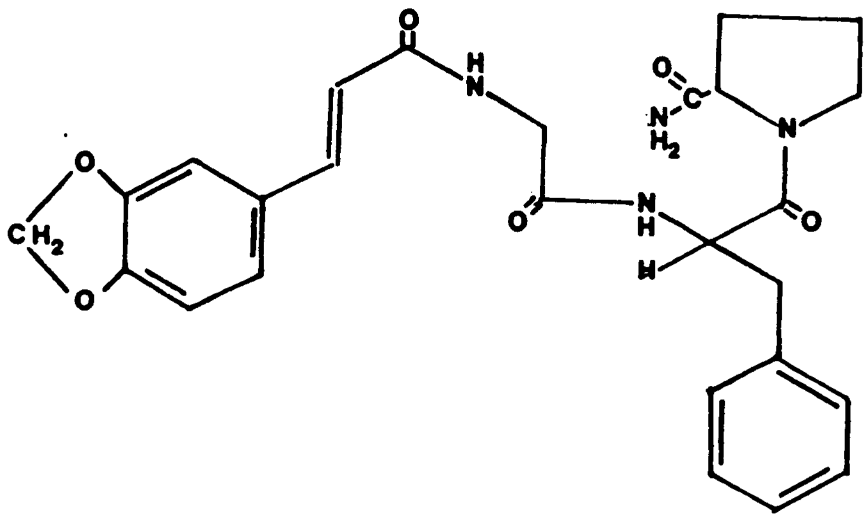

(VIII)

Le produit est synthétisé en utilisant le même procédé général que celui décrit précédemment pour les analogues cinnamoyl et 4-fluorocinnamoyl.

1. Ester méthylique de 3,4-méthylènedioxycinnamoyl-glycine

On réalise le couplage de l'acide 3,4-méthylènedioxycinnamique (15,0 g) avec le chlorhydrate de l'ester méthylique de glycine (9,78g).

| | |
|---|---|
| Rendement | 12.2 g |
| CCM | ($CHCl_3$:MeOH:AcOH,45:4:1)$R_f$ = 0,6 |

2. 3,4-méthylènedioxycinnamoyl-glycine

Le produit de l'étape précédente (12,2 g) est saponifié comme décrit précédemment.

| | |
|---|---|
| Rendement | 7,2 g |
| CCM | ($CHCl_3$:MeOH:AcOH, 45:4:1): $R_f$ = 0,2 (n-BuOH:AcOH:$H_2$O, 4:1:1) |

3. Le 3,4 méthylènedioxycinnamoyl-glycine-L-phényl alanyl-L-prolinamide

On réalise le couplage du produit de l'étape précédente (3,9 g) avec du trifluoroacétate de L-phénylalanyl-L-prolinamide (5,55 g) ce qui conduit au produit recherché.

| | |
|---|---|
| Rendement | 1,3 g |
| CCM | ($CHCl_3$:MeOH:AcOH, 45:4:1): $R_f$ = 0,5 |
| PF | 92 - 93 ° C (dec.) |

EXEMPLE 4 : Synthèse du 3,4,5-triméthoxycinnamoyl-glycyl-L-phénylalanyl-L-prolinamide de formule IX

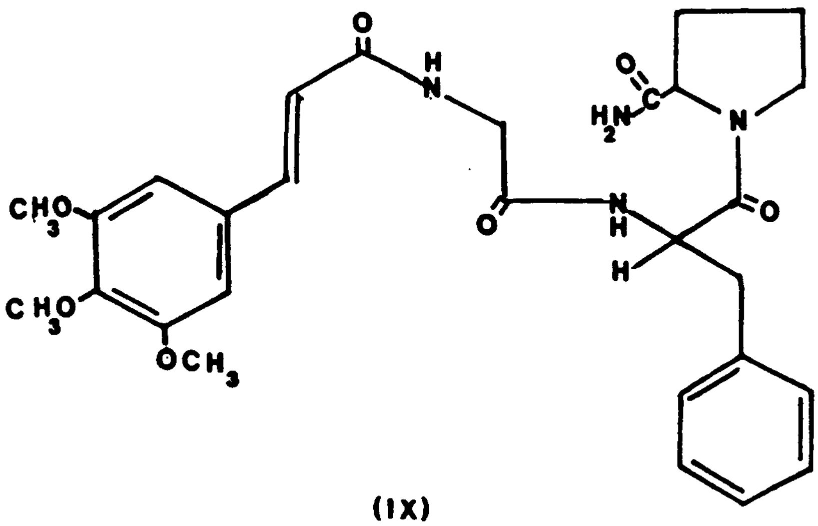

(IX)

On utilise les méthodes générales décrites ci-dessus pour la synthèse du 4-fluorocinnamoyl-glycyl-L -phénylalanyl-L-prolinamide et l'analogue cinnamoyl.

1. Ester méthylique de 3,4,5-triméthoxycinnamoyl-glycine

On réalise le couplage de l'acide 3,4,5-triméthoxycinnamique (15,0 g) avec le chlorhydrate de l'ester méthylique de glycine (7,9 g).

| | |
|---|---|
| Rendement | 13,2 g |
| CCM | ($CHCl_3$:MeOH:AcOH, 45:4:1):$R_f$ = 0,7 |

2. 3,4,5-triméthoxycinnamoyl-glycine

Le produit de l'étape précédente (13,2 g) est. soumis à une opération de saponification comme décrit précédemment.

| | |
|---|---|
| Rendement | 5,8 g |
| CCM | ($CHCl_3$:MeOH:AcOH, 45:4:1): $R_f$ = 0,2<br>(n-BuOH:AcOH:$H_2O$,4:1:1): $R_f$ = 0,6 |

3. 3,4,5-triméthoxycinnamoyl-glycyl-L-phénylalanyl-L-prolinamide

Le produit de l'étape précédente (3,0 g) est couplé avec le trifluoroacétate de L-phénylalanyl-L-prolinamide (4,0 g) pour obtenir le produit recherché.

| | |
|---|---|
| Rendement | 2,3 g |
| CCM | ($CHCl_3$:MeOH:AcOH, 45:4:1): $R_f$ = 0,7 |
| PF | 122 °C |

EXEMPLE 5 : Synthèse du cinnamoyl-glycyl-L-leucyl-L-prolinamide de formule X

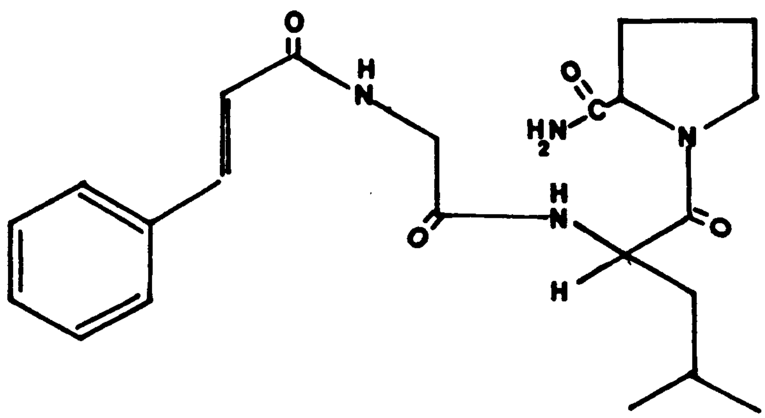

(X)

1. t-butyloxycarbonyl-L-leucyl-L-prolinamide

En suivant le procédé général décrit précédemment pour la synthèse du t-butyloxycarbonyl-L-phenylalanyl-L-prolinamide, on réalise l'activation d'une solution de t-butyloxycarbonyl-L-leucine (32,64 g), dans du tétrahydrofurane par traitement avec de la N-méthylmorpholine (18,22 ml), suivie de chloroformate d'isobutyle (16,99 ml) à -5 °C. Après 15 minutes, on ajoute le chlorhydrate de L-prolinamide (15,0 g) en solution dans du diméthyformamide, puis de la N-méthylmorpholine (18,22 ml) et on soumet le mélange à agitation durant environ 14 heures à température ambiante. Le produit résultant est utilisé sans autre purification pour l'étape de synthèse suivante.

| | |
|---|---|
| Rendement | 30,0 g |
| CCM | ($CHCl_3$:MeOH:AcOH, 85:10:5): $R_f$ = 0,6 |

2. Trifluoroacétate-L-leucyl-L-prolinamide

On élimine les groupements protecteurs du produit de l'étape précédente (30,0 g) comme décrit précédemment par traitement avec de l'acide trifluoroacétique et du chlorure de méthylène (1:1, 150 ml) à température ambiante. Le mélange réactionnel est soumis à évaporation et trituré avec de l'éther pour donner le produit recherché qui est utilisé sans autre purification dans l'étape de synthèse suivante.

| | |
|---|---|
| Rendement | 29,5 g |
| CCM | (n-BuOH:AcOH:$H_2O$, 4:1:1): $R_f$ = 0,6<br>($CHCl_3$:MeOH:AcOH, 85:15:5): $R_f$ = 0,2 |

3. Ester-méthylique de cinnamoyl-glycine

On réalise le couplage de l'acide cinnamique (38,0 g) au chlorhydrate de l'ester méthylique de la glycine dans du diméthylformamide par traitement avec du dicyclohexylcarbodiimide (52 , 84 g) et du 1-hydroxybenzotriazole (39,24 g) suivi de N-méthylmorpholine (37,56ml). Le mélange réactionnel est soumis à agitation durant 14 h à température ambiante et l'on récupère le produit recherché.

| | |
|---|---|
| Rendement<br>CCM | 34,5 g<br>($CHCl_3$:MeOH:AcOH, 85:15:5): $R_f$ = 0,8 |

4. Cinnamoyl-glycine

On soumet le produit de l'étape précédente (35,0 g) à une saponification par traitement avec de l'hydroxide de sodium (7,4 g) dans du méthanol pendant 1h à température ambiante.

On récupère le produit recherché qu'on utilise tel quel dans l'étape suivante.

| | |
|---|---|
| Rendement | 30,2 g |
| CCM | (n-BuOH:AcOH:$H_2O$, 4:1:1): $R_f$ = 0,4<br>($CHCl_3$:MeOH:AcOH, 85:15:5): $R_f$ = 0,8 |

5. Cinnamoyl-glycyl-L-leucyl-L-prolinamide

On couple le produit de l'étape précédente (15,0g) avec du trifluoroacétate-L-leucyl-L-prolinamide (24,42 g) dans du diméthylformamide en utilisant du dicyclohexylcarbodiimide (15,05 g) et du 1-hydroxybenzotriazole (11,2 g) suivi de L-méthylmorpholine (7,37 ml). Après environ 14 h, on récupère le produit du mélange réactionnel sous forme de solide blanc.

| | |
|---|---|
| Rendement<br>CCM<br>PF | 5,8 g<br>($CHCl_3$:MeOH:AcOH, 85:15:5): $R_f$ = 0,6<br>107,5 - 116,5 ° C |

EXEMPLE 6 : Synthèse du benzoyl-glycyl-L-phénylalanyl -L-prolinamide de formule XI

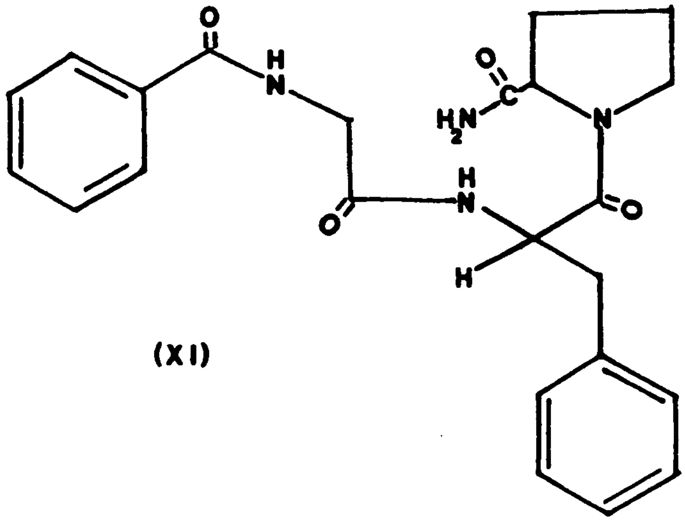

(XI)

On opère comme décrit précédemment pour les mêmes types de produit.

1. Ester t-butylique de benzoyl-glycine

En utilisant le procédé général décrit précédemment pour la synthèse de l'ester méthylique de cinnamoyl-glycine, on dissout le chlorhydrate de l'ester t-butylique de glycine (10,88 g) dans du diméthylformamide (environ 100ml), on refroidit le mélange réactionnel à 0 ° C puis on le traite avec de la N-méthylmorpholine (9,04 ml, 1 équivalent) suivie immédiatement par une solution pré-refroidie d'acide benzoique (7,94 g) dans de l'acétate d' éthyle (environ 75ml) qui a été préalablement pré-activé par traitement avec de la N-méthylmorpholine (9,04 ml) suivi de chloroformate d'isobutyle (8,4 ml) à -20 ° C.

On obtient l'ester t-butylique de benzoyle-glycine sous forme d'un solide blanc utilisé pour l'étape suivante sans autre purification.

| | |
|---|---|
| Rendement | 8,1 g |
| CCM | ($CHCl_3$:MeOH:AcOH, 85:10:5): Rf = 0,9 |

2. Benzoyl-glycine

Le produit de l'étape précédente (8,1 g) est traité a l'aide d'acide trifluoroacétique (90 ml) pendant 1 h à température ambiante. Après évaporation de l'acide trifluoroacétique et trituration du résidu avec de l'éther, on obtient un produit sous forme d'un solide blanc utilisé sans autre purification.

| | |
|---|---|
| Rendement | 5,2 g |
| CCM | ($CHCl_3$:MeOH:AcOH, 85:10:5): Rf = 0,35 |
| PF | 181 - 184 ° C |

3. Benzoyl-glycyl-L-phénylalanyl-L-prolinamide

Le produit de l'étape précédente (2,61 g sous forme de solution dans le diméthylformamide) est couplé à du trifluroroacétate de L-phénylalanyl-L-prolinamide (5,5 g sous forme de solution dans le tétrahydrofurane) par la méthode utilisant l'anhydride mixte. Le produit obtenu se présente sous forme d'un solide blanc.

| | |
|---|---|
| Rendement | 1,67 g |
| CCM | ($CHCl_3$:MeOH:AcOH, 85:10:5): Rf = 0,6 |
| PF | 95 - 113 °C |

EXEMPLE 7 : Synthèse du phénylacétyl-glycyl-L-phénylalanyl-L-prolinamide de formule XII

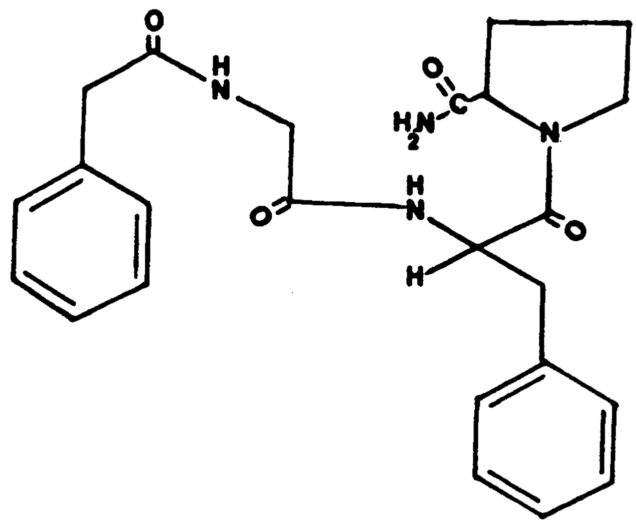

(XII)

1. Ester t-butylique de phénylacétyl-glycine

En opérant selon le procédé décrit ci-dessus pour la synthèse de l'ester t-butylique de benzoyl-glycine, on active une solution d'acide phénylacétique (11,44 g) dans de l'acétate d'éthyle en utilisant la méthode à l'anhydride mixte et en mettant en oeuvre de la N-méthylmorpholine (11,66 ml) suivie de chloroformate d'isobutyle (10,89 ml) à -20 °C et on couple avec le chlorhydrate de l'ester t-butylique de glycine (14,04g) dans du chlorure de méthylène (150 ml) qui a été neutralisé par addition de N-méthylmorpholine (11,66 ml).

Le produit obtenu se présente sous forme d'une huile qui est utilisée dans l'étape suivante sans autre purification.

2. Phénylacétyl-glycine

Le produit de l'étape précédente est traité avec de l'acide trifluoroacétique (250 ml) pendant 1 h à température ambiante. L'évaporation du mélange réactionnel et la trituration du résidu avec de l'éther conduit a l'obtention du produit désiré qui se présente sous forme d'un solide blanc.

| | |
|---|---|
| Rendement | 6,8 g |
| CCM | ($CHCl_3$:MeOH:AcOH, 85:10:5): Rf = 0,25 |
| PF | 132 - 135 °C |

3. Phénylacétyl-glycine-L-phénylalanyl-L-prolinamide

En opérant comme décrit plus haut, on réalise le couplage de la phénylacétyl-glycine (4 g sous forme de solution dans le tétrahydrofurane) avec le trifluoroacétate de L-phénylalanyl-L-prolinamide (7,87 g sous forme de solution dans le chlorure de méthylène) en utilisant la méthode à l'anhydride mixte. Le produit obtenu se présente sous forme d'un solide blanc.

| | |
|---|---|
| Rendement | 5,05 g |
| CCM | ($CHCl_3$:MeOH:AcOH, 85:10:5): Rf = 0,7 |
| PF | 81 - 98 °C |

EXEMPLE 8 :

Préparation du dérivé de L-proline de formule XIII

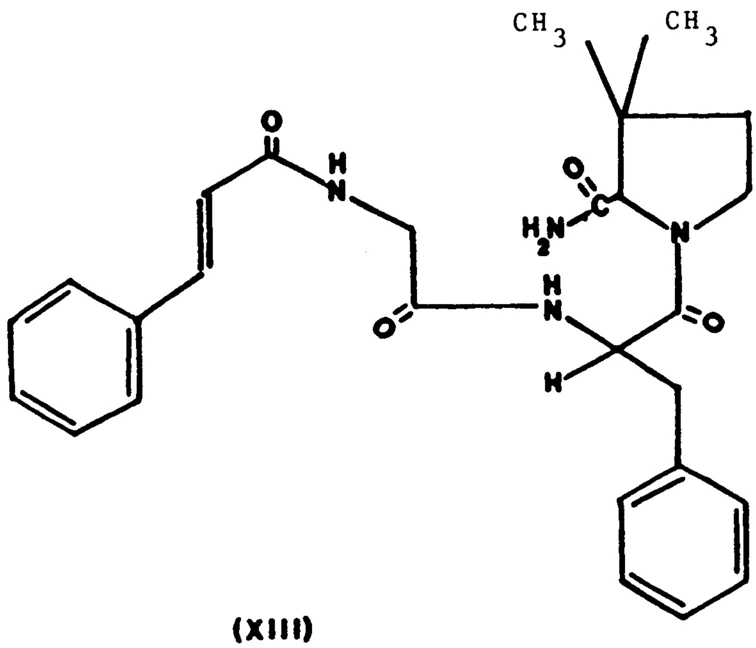

(XIII)

On opère comme décrit dans l'exemple 1 mais en utilisant le diméthyl prolineamide. Ce dérivé est synthétisé en adaptant la méthode du brevet UK 1.523598 concernant le L-pyroglutamoyl-L-histidyl-L-3,3 diméthylprolineamide.

EXEMPLE 9 : Etudes pharmacologigues :

1/ Test d'Irwin :

On administre à des souris mâles de souche NMRI, par voie i.p., 0,25 ml/20 g de poids corporel d'une suspension dans 5% de gomme arabique de produit de l'invention.

Les animaux témoins reçoivent uniquement la suspension à 5% de gomme arabique.

Les modifications du comportement, les symptômes de neurotoxicité, le diamètre de la pupille et la température rectale sont enregistrés selon la grille d'observation standardisée, selon Irwin dans Psychopharmacologia, 1968, 13, 222-257.

Ces observations sont effectuées 15, 30, 60, 120 et 180 minutes après l'injection et 24 heures plus tard.

Les produits de l'invention ont été étudiés à des doses de 1024, 512 et 256 mg.kg-1.

Aux doses étudiées, on n'observe ni mort, ni convulsions des animaux.

Avec le composé de formule VI, on remarque une légère sédation (1souris/3) ou excitation (1souris/3) entre 1 et 2 heures après l'administration de 1024 mg-kg-1, avec une ptose modérée (3 souris/3) et une hypothermie (2 souris/3).

A la dose de 512 mg.kg-1, le composé de formule VI ne provoque qu'une légère hypothermie. A 256 mg.kg-1, aucun changement n'est noté par rapport aux témoins.

2/ Test d'évitement passif :

a) - amnésie induite par la scopolamine :

On opère comme décrit par Glick et Zimmerberg dans Behavioral Biology, 1972 7 : 245-254 et par Lenègre et al dans Pharmacol. Biochem. Behav. 29 (3), 1988.

On introduit la souris dans le compartiment éclairé d'une boîte à deux compartiments. Lorsqu'elle traverse vers le compartiment le plus sombre, elle reçoit une impulsion électrique aux pattes de 0,3 mA jusqu'à ce qu'elle retourne dans le compartiment éclairé (essai 1).

Lorsqu'on remet la souris 24 heures plus tard dans la boîte (essai 2), elle évite d'entrer dans le compartiment le plus sombre. Une injection, par voie i.p. , de scopolamine (1 mg.kg-1) 30 minutes avant l'essai 1 réduit la mémoire comme le montre le délai de réaction de la souris pour traverser vers le compartiment le plus sombre à l'essai 2.

Les produits de l'invention sont testés aux doses suivantes : 0,25, 1, 4, 8 et 16 mg.kg-1 et administrés, par voie i.p. ou orale, 60 minutes avant S1.

On utilise le piracetam comme composé de référence.

Les résultats obtenus avec les composés des, exemples montrent que ces produits, administrés par voie i.p. ou orale 60 minutes avant l'essai, ne provoquent, pas de changement dans les latences pour traverser vers le compartiment sombre ou pour s'échapper de ce compartiment durant l'essai 1.

Le piracetam, dans les mêmes conditions d'expérience, ne produit pas d'effet sur les deux paramètres mesurés durant l'essai 1.

Pendant l'essai 2, on constate que la scopolamine, administrée 30 minutes avant l'essai 1, provoque une diminution du temps pour traverser vers le compartiment sombre.

Le produit de l'exemple 1, administré par voie i.p. 60 minutes avant l'essai 1, provoque une réaction antagoniste vis-à-vis des effets de la scopolamine aux doses de 1,4 et 16 mg/kg, l'effet étant significatif à la dose de 1 mg/kg.

Dans la même expérience, le piracétam provoque un antagonisme de l'amnésie induite par la scopolamine, mais à une dose de 512 mg/kg, ce qui met en évidence les propriétés avantageuses des produits de l'invention actifs à moindre dose.

De même, des essais réalisés par administration des produits de l'invention par voie orale ont montré qu'ils exercent un fort antagonisme vis-à-vis de l'amnésie induite par la scopolamine par rapport au produit connu de l'état de la technique.

Les produits des exemples 1, 2, 3, 6 et 7 sont actifs sur ce test à 2,8 et 32 mg/kg. Cette activité est significative dès la dose de 2 mg/kg. L' intérêt des produits de l'invention est souligné par le fait que le piracétam est actif sur ce test mais à une dose nettement supérieure de 2048 mg/kg.

b) - Amnésie induite par le diazépam :

On administre à des souris de souche NMRI 0,25 ml/20 g de poids corporel du produit de l'exemple 1 (suspension dans 5% de gomme arabique) ou, à titre de composé de référence, d'une solution aqueuse de piracétam, ou encore de diazépam (suspension à 5% dans gomme arabique). Les animaux témoins ne reçoivent que des injections de véhicule (suspension de gomme arabique).

On réalise le test comme indiqué ci-dessus, lorsqu'on induit l'amnésie par injection de scopolamine, mais en remplaçant cette dernière par du diazépam. Une injection par voie i.p. de diazépam 30 minutes avant l'essai 1, à une dose de 1 mg.kg-1 réduit de manière significative la mémoire, comme le montre la diminution du temps de réaction chez la souris pour traverser le compartiment sombre durant l'essai 2.

Une réduction importante de l'amnésie à dose moindre de produit est observée avec les produits de l'invention.

Ainsi, on constate que le produit de l'exemple 1 étudié aux doses de 0,25, 1 et 4 mg.kg-1, et administré 60 minutes avant l'essai 1, exerce un effet antagoniste, dépendant de la dose, vis-à-vis du diazépam.

Cet effet apparaît statistiquement significatif en administrant 4 mg.kg-1 de produit.

L'amnésie est réduite par des agents à action nootropique tels que le piracétam ou ses dérivés, utilisé dans ces essais mais à une dose de 512 mg.kg-1, par voie ip., 60 minutes avant l'essai 1.

Ces résultats montrent que les produits de l'invention corrigent, à faible dose, l'amnésie induite aussi bien par la scopolamine que par le diazépam, en opérant dans des conditions expérimentales similaires.

3/ Antagonisme du sommeil barbiturique

On administre à des souris de souche NMRI un produit de l'invention par voie orale aux doses de 2,8 et 32 mg.kg-1, une heure avant l'administration intrapéritonéale de barbital à la dose de 50 mg/kg.

Le produit de l'exemple 1 diminue significativement, aux doses de 2 et 8 mg/kg, la durée du sommeil par rapport au groupe témoin.

4/ Potentialisation des effets de la L-dopa

On administre à des souris NMRI un produit de l'invention par voie orale aux doses de 2,8 et 32 mg/kg une heure avant l'administration par voie intrapéritonéale de 150 mg/kg de L-dopa. Cette dose de L-dopa ne provoque pas de modification comportementale chez le groupe témoin. Aux doses de 8 et 32 mg/kg, le produit de l'exemple 1 fait apparaître chez les animaux traités des modifications du comportement tels que course rapide et sauts.

L'ensemble de ces résultats met en évidence les propriétés avantageuses des produits de l'invention, plus spécialement en tant que principes actifs de médicaments à effet nootrope.

5/ Etude de la sécrétion des hormones adénohypophysaires

Des rats mâles reçoivent 2 mg/kg, 8 mg/kg ou 32 mg/kg IP du produit de l'exemple 1 en suspension dans la gomme arabique (2,5%) et 32 mg/kg IP des produits des exemples 2 et 3. Les rats sont sacrifiés par décapitation 30 minutes après et une aliquote des échantillons de plasma est conservée par congélation (-20 °C) jusqu'au dosage. Avant l'expérience, les rats sont habitués à la manipulation afin d'éviter tout effet de stress.

Prolactine, GH et TSH sont dosées par radio-immuno-essai.

Le produit de l'exemple 1 augmente légèrement, mais en fonction de la dose, les taux de prolactine augmentent très légèrement les taux de TSH à 8 mg/kg. Les produits des exemples 2 et 3 n'ont aucune activité à 32 mg/kg sur les taux de prolactine, TSH et GH.

## Revendications

**Revendications pour les Etats contractants suivants : AT, BE, CH, DE, ES, FR, GB, IT, LI, LU, NL, SE**

**1.** Dérives de la L-proline, caractérisés ce qu'ils répondent à la formule I :

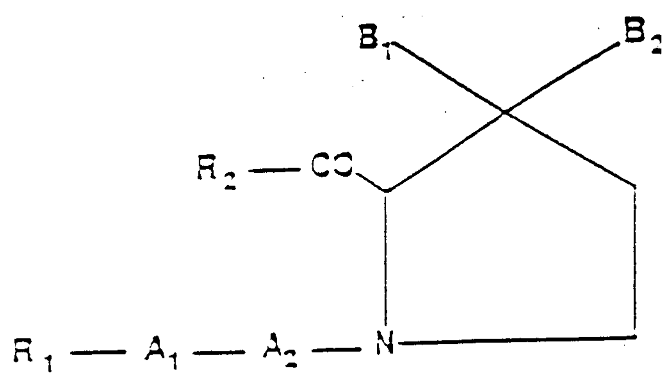

(I)

dans laquelle

- R₁ est un groupe de formule II

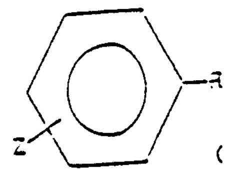

(II)

dans laquelle R est un radical carbonyle CO-, un radical acyle Y-CO- ou un radical oxy-acyle O-Y-CO-, dans lesquels Y est une chaîne alcoyle ou alcényle, notamment de 1 à 4 atomes de carbone, Z représente un ou plusieurs atomes d'hydrogène, ou un ou plusieurs substituants en position ortho et/ou ortho' et/ou méta et/ou méta' et/ou para, choisi(s) parmi les atomes d'halogène, un groupe $CF_3$, les radicaux alcoyle ou alcoxy de 1 à 4 atomes de carbone, et pour deux substituants voisins, un groupe alcoylènedioxy, le groupe alcoylène renfermant de 1 à 3 atomes de carbone.

- $R_2$ est un radical $NH_2$ ou OH, ou des dérivés fonctionnels de ces radicaux,
- $A_1$ et $A_2$, identiques ou différents l'un de l'autre, sont des résidus d'acides aminés, et
- $B_1$ et $B_2$, identiques ou différents l'un de l'autre, représentent un atome d'hydrogène ou un groupe méthyle, et les sels pharmacologiquement acceptables de ces dérivés, sous réserve que d'une part $A_1$ soit différent d'un résidu phénylalanyle lorsque $B_1$ et $B_2$ représentent un atome d'hydrogène, $R_2$ représente un goupe -OH, $R_1$ représente un radical benzoyle et $A_2$ est un résidu alanyle, et d'autre part que $A_2$ soit différent d'un résidu proline, lorsque R représente un groupe -CO ou -YCO dans lequel Y est un groupe alcoyle, $B_1$ et $B_2$ représentent un atome d'hydrogène et $R_2$ est un groupe -OH ou $-NH_2$ ou leurs dérivés fonctionnels.

**2.** Dérivés selon la revendication 1, caractérisés en ce que Z, dans la formule II, est un atome d'hydrogène.

**3.** Dérivés selon la revendication 1, caractérisés en ce que Z, dans la formule II, est un atome d'halogène choisi parmi le chlore ou le fluor, un groupe $CF_3$, un radical alcoxy choisi parmi un groupe méthoxy ou éthoxy, et pour deux positions voisines, un radical alcoylène-dioxy choisi parmi le 3,4-méthylène ou 3,4-èthylène dioxy.

**4.** Dérivés selon l'une quelconque des revendications 1 à 3, caractérisés en ce que dans la formule II, R est choisi parmi les groupes CO-, $CH_2$-CO-, $CH_2$-$CH_2$-CO-, $CH_2$-$CH_2$-$CH_2$-CO-, CH=CH-CO- ou O-$CH_2$-CO-.

**5.** Dérivés selon la revendication 1, caractérisés en ce que $R_1$ est un groupe cinnamoyle, le cas échéant substitué.

**6.** Dérivés selon l'une quelconque des revendications 1 à 5, caractérisés en ce que $A_1$ et $A_2$ sont des acides aminés naturels.

**7.** Dérivés selon la revendication 6, caractérisés en ce que $A_1$ est un résidu glycyle.

**8.** Dérivés selon la revendication 6, caractérisés en ce que $A_1$ est choisi parmi les résidus L-alanyle et L-valyle.

**9.** Dérivés selon la revendication 6, caractérisés en ce que $A_1$ est choisi parmi les résidus L-glycyle, L-alanyle et L-valyle, et $A_2$ parmi les résidus glycyle, L-phénylalanyle, L-histidyle, L-leucyle, L-valyle et L-alanyle.

**10.** Dérivés de L-proline, choisis parmi les cinnamoyl-glycyl-L-phénylalanyl-L-prolinamide, 4-fluorocinnamoyl -glycyl-L-phénylalanyl-L-prolinamide, 3,4-méthylènedioxy-cinnamoyl-glycyl-L-prolinamide, 3,4,5-triméthoxycinnamoylglycyl-L-phénylalanyl-L-prolinamide, cinnamoyl-glycyl-L-leucyl-L-prolinamide, benzoyl-glycyl-L-phénylalanyl-L-prolinamide, phénylacétyl-glycyl-L-phénylalanyl-L-prolinamide et cinnamoyl-glycyl-L-phénylalanyl-L-3,3'diméthyl-prolinamide.

**11.** Composition pharmaceutique, caractérisée en ce qu'elle renferme une quantité efficace d'au moins un dérivé de L-proline selon l'une quelconque des revendications 1 à 10, en association avec un véhicule pharmaceutique.

**12.** Composition selon la revendication 11, caractérisée en ce qu'elle est administrable par voie orale, rectable, nasale, injectable.

**13.** Composition selon la revendication 11, caractérisée en ce qu'il s'agit de tablettes, comprimés, gélules, gouttes, pilules, liposomes et qu'elle renferme de 1 à 100 mg de principe actif par unité de prise, de préférence de 2,5 à 50 mg.

**14.** Composition selon la revendication 11, caractérisée en ce qu'il s'agit de solutions injectables, ces solutions renfermant avantageusement, par unité de prise, de 1 à 50 mg de dérivé de L-proline, de préférence de 0,5 à 50 mg.

**15.** Réactif biologique, caractérisé en ce qu'il est élaboré à partir d'un dérivé de L-proline selon l'une quelconque des revendications 1 à 10.

**16.** Procédé de préparation de dérivés de L-proline selon la revendication 1, caractérisé par le fait qu'on fait réagir

- un dérivé de formule III :

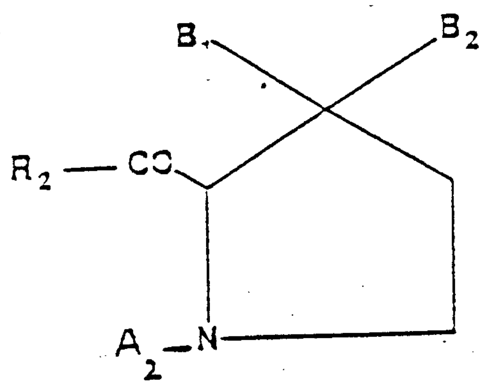

(III)

- avec un dérivé de formule IV

$R_1 - A_1$ (IV)

dans lesquelles $B_1$, $B_2$, $A_1$, $A_2$, $R_1$ et $R_2$ sont tels que définis dans la revendication 1.

**17.** Procédé selon la revendication 16, caractérisé en ce que pour obtenir le dérivé de formule III, on met en oeuvre un dérivé de proline de formule V :

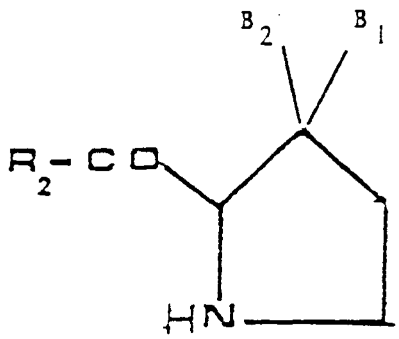

(V)

et un acide aminé $A_2$,
$B_1$, $B_2$, $R_2$ et $A_2$ étant tels que définis dans la revendication 1.

**18.** Procédé selon la revendication 16 ou 17, caractérisé en ce que le dérivé $R_1$-$A_1$, de formule IV est obtenu par condensation de dérivés réactifs de $R_1$ et de $A_1$, la fonction carboxyle de $A_1$ étant bloquée par un groupe protecteur, ladite condensation étant suivie d'une saponification à l'aide d'une base forte.

**Revendications pour l'Etat contractant suivant : ES**

**1.** Procédé de préparation de dérivés de L-proline, caractérisé par le fait qu'on fait réagir

- un dérivé de formule III :

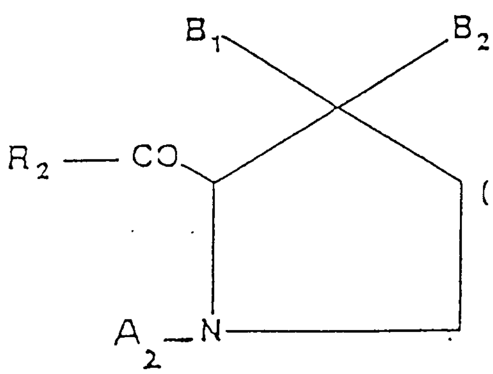

(III)

- avec un dérivé de formule IV

$R_1$ - $A_1$ (IV)

ce qui conduit à l'obtention de dérivés de proline de formule I :

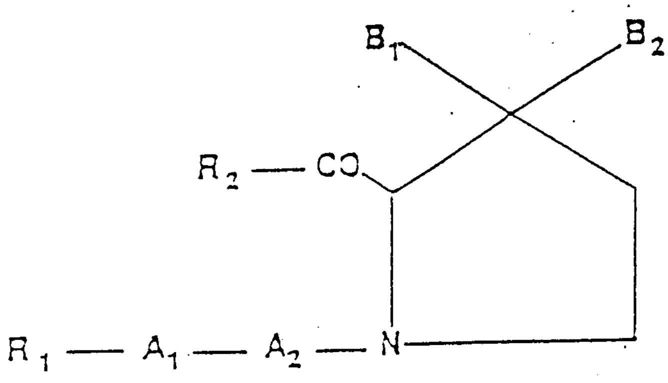

(I)

les substituants ayant dans ces formules les significations suivantes :

- R1 est un groupe de formule II

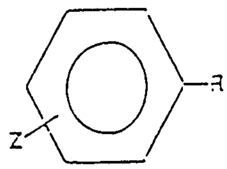

(II)

dans laquelle R est un radical carbonyle CO-, un radical acyle Y-CO- ou un radical oxy-acyle O-Y-CO-, dans lesquels Y est une chaîne alcoyle ou alcényle, notamment de 1 à 4 atomes de carbone, Z représente un ou plusieurs atomes d'hydrogène, ou un ou plusieurs substituants en position ortho et/ou ortho' et/ou méta et/ou méta' et/ou para, choisi(s) parmi les atomes d'halogène, un groupe $CF_3$, les radicaux alcoyle ou alcoxy de 1 à 4 atomes de carbone, et pour deux substituants voisins, un groupe alcoylènedioxy, le groupe alcoylène renfermant de 1 à 3 atomes de carbone.

- $R_2$ est un radical $NH_2$ ou OH, ou des dérivés fonctionnels de ces radicaux,
- $A_1$ et $A_2$, identiques ou différents l'un de l'autre, sont des résidus d'acides aminés, et
- $B_1$ et $B_2$, identiques ou différents l'un de l'autre, représentent un atome d'hydrogène ou un groupe méthyle, et les sels pharmacologiquement acceptables de ces dérivés, sous réserve que d'une part $A_1$ soit différent d'un résidu phénylalanyle lorsque $B_1$ et $B_2$ représentent un atome d'hydrogène, $R_2$ représente un groupe -OH, $R_1$ représente un radical benzoyle et $A_2$ est un résidu alanyle, et d'autre part que $A_2$ soit différent d'un résidu proline, lorsque R représente un groupe -CO ou -YCO dans lequel Y est un groupe alcoyle, $B_1$ et $B_2$ représentent un atome d'hydrogène et $R_2$ est un groupe -OH ou -$NH_2$ ou leurs dérivés fonctionnels.

**2.** Procédé selon la revendication 1, caractérisé en ce que Z, dans la formule II, est un atome d'hydrogène.

**3.** Procédé selon la revendication 1, caractérisé en ce que Z, dans la formule II, est un atome d'halogène choisi parmi le chlore ou le fluor, un groupe $CF_3$, un radical alcoxy choisi parmi un groupe méthoxy ou éthoxy, et pour deux positions voisines, un radical alcoylène-dioxy choisi parmi le 3,4-méthylène ou 3,4-éthylène dioxy.

**4.** Procédé selon l'une quelconque des revendications 1 à 3, caractérisé en ce que dans la formule II, R est choisi parmi les groupes CO-, $CH_2$-CO-, $CH_2$-$CH_2$-CO, $CH_2$-$CH_2$-$CH_2$-CO-, CH = CH-CO- ou O-$CH_2$-CO-.

**5.** Procédé selon la revendication 1, caractérisé en ce que $R_1$ est un groupe cinnamoyle, le cas échéant substitué.

**6.** Procédé selon l'une quelconque des revendications 1 à 5, caractérisé en ce que $A_1$ et $A_2$ sont des acides aminés naturels.

**7.** Procédé selon la revendication 6, caractérisé en ce que $A_1$ est un résidu glycyle.

**8.** Procédé selon la revendication 6, caractérisé en ce que $A_1$ est choisi parmi les résidus L-alanyle et L-valyle.

**9.** Procédé selon la revendication 6, caractérisé en ce que $A_1$ est choisi parmi les résidus L-glycyle, L-alanyle et L-valyle, et $A_2$ parmi les résidus glycyle, L-phénylalanyle, L-histidyle, L-leucyle, L-valyle et L-alanyle.

**10.** Procédé selon la revendication 1, caractérisé en ce qu'on obtient des produits de formule I choisis parmi les cinnamoyl-glycyl-L-phénylalanyl-L-prolinamide, 4-fluro-cinnamoyl-glycyl-L-phénylalanyl-L-prolinamide, 3,4-méthy-lènedioxy-cinnamoyl-glycyl-L-prolinamide, 3,4,5-triméthoxy-cinnamoylglycyl-L-phénylalanyl-L-prolinamide, cinnamoyl-glycyl-L-leucyl-L-prolinamide, benzoyl-glycyl-L-phényla-lanyl-L-prolinamide-phénylacétyl-glycyl-L-phénylalanyl-L-prolinamide et cinnamoyl-glycyl-L-phénylalanyl-L-3,3' diméthyl-prolinamide.

**11.** Procédé selon l'une des revendications 1 à 10, caractérisé en ce que pour obtenir le dérivé de formule III, on met en oeuvre un dérivé de proline de formule V :

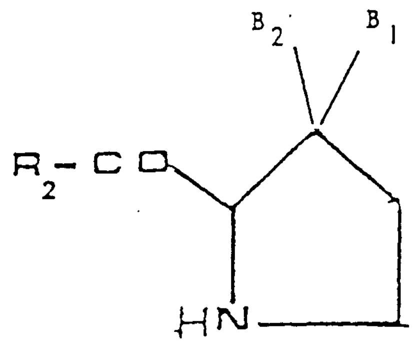

(V)

et un acide aminé $A_2$,
$B_1$, $B_2$, $R_2$ et $A_2$ étant tels que définis dans la revendication 1.

**12.** Procédé selon l'une des revendications précédentes, caractérisé en ce que le dérivé $R_1$-$A_1$, de formule IV est obtenu par condensation de dérivés actifs de $R_1$ et de $A_1$, la fonction carboxyle de $A_1$ étant bloquée par un groupe protecteur, ladite condensation étatn suivie d'une saponification à l'aide d'une base forte.

**13.** Procédé de préparation de compositions pharmaceutiques, caractérisé en ce qu'on mélange une quantité efficace d'au moins un dérivé de L-proline de formule I, selon la revendication 1 ou leurs sels, avec un véhicule pharmaceutique.

**14.** Procédé selon la revendication 13, caractérisé en ce que le véhicule pharmaceutique est choisi pour une administration par voie orale, rectale, nasale, injectable.

**15.** Procédé selon la revendication 13, caractérisé en ce qu'on forme des tablettes, comprimés, gélules, gouttes, pilules, liposomes renfermant de 1 à 100 mg de principe actif par unité de prise, de préférence de 2,5 à 50 mg.

**16.** Procédé selon la revendication 13, caractérisé en ce qu'on forme des solutions injectables, ces solutions renfermant avantageusement, par unité de prise de 1 à 50 mg de dérivé de L-proline, de préférence de 0,5 à 50 mg.

**17.** Utilisation des dérivés de L-proline obtenus selon l'une des revendications 1 à 42 comme réactif biologique.

**Revendications pour l'Etat contractant suivant : GR**

**1.** Dérivés de la L-proline, caractérisés en ce qu'ils répondent à la formule I :

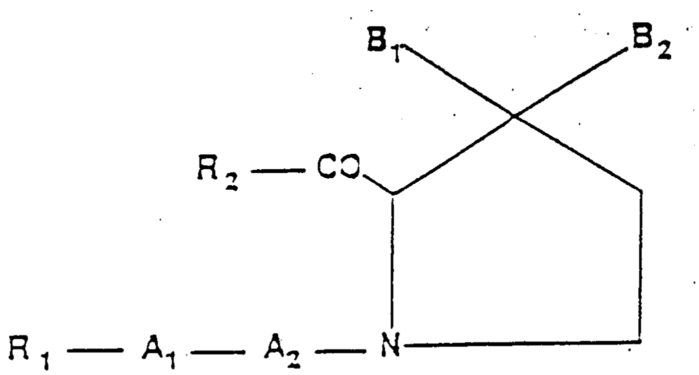

(I)

dans laquelle

- R1 est un groupe de formule II

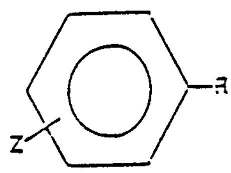

(II)

dans laquelle R est un radical carbonyle CO-, un radical acyle Y-CO- ou un radical oxy-acyle O-Y-CO-, dans lesquels Y est une chaîne alcoyle ou alcényle, notamment de 1 à 4 atomes de carbone, Z représente un ou plusieurs atomes d'hydrogène, ou un ou plusieurs substituants en position ortho et/ou ortho' et/ou méta et/ou méta' et/ou para, choisi(s) parmi les atomes d'halogène, un groupe $CF_3$, les radicaux alcoyle ou alcoxy de 1 à 4 atomes de carbone, et pour deux substituants voisins, un groupe alcoylènedioxy, le groupe alcoylène renfermant de 1 à 3 atomes de carbone.

- $R_2$ est un radical $NH_2$ ou OH, ou des dérivés fonctionnels de ces radicaux,
- $A_1$ et $A_2$, identiques ou différents l'un de l'autre, sont des résidus d'acides aminés, et
- $B_1$ et $B_2$, identiques ou différents l'un de l'autre, représentent un atome d'hydrogène ou un groupe méthyle, et les sels pharmacologiquement acceptables de ces dérivés, sous réserve que d'une part $A_1$ soit différent d'un résidu phénylalanyle lorsque $B_1$ et $B_2$ représentent un atome d'hydrogène, $R_2$ représente un groupe -OH, $R_1$ représente un radical benzoyle et $A_2$ est un résidu alanyle, et d'autre part que $A_2$ soit différent d'un résidu proline, lorsque R représente un groupe -CO ou -YCO dans lequel Y est un groupe alcoyle, $B_1$ et $B_2$ représentent un atome d'hydrogène et $R_2$ est un groupe -OH ou $-NH_2$ ou leurs dérivés fonctionnels.

**2.** Dérivés selon la revendication 1, caractérisés en ce que Z, dans la formule II, est un atome d'hydrogène.

**3.** Dérivés selon la revendication 1, caractérisés en ce que Z, dans la formule II, est un atome d'halogène choisi parmi le chlore ou le fluor, un groupe $CF_3$, un radical alcoxy choisi parmi un groupe méthoxy ou éthoxy, et pour deux positions voisines, un radical alcoylène-dioxy choisi parmi le 3,4-méthylène ou 3,4-éthylène dioxy.

**4.** Dérivés selon l'une quelconque des revendications 1 à 3, caractérisés en ce que dans la formule II, R est choisi parmi les groupes CO-, $CH_2$-CO-, $CH_2$-$CH_2$-CO, $CH_2$-$CH_2$-$CH_2$-CO-, CH=CH-CO- ou O-$CH_2$-CO-.

**5.** Dérivés selon la revendication 1, caractérisés en ce que $R_1$ est un groupe cinnamoyle, le cas échéant substitué.

**6.** Dérivés selon l'une quelconque des revendications 1 à 5, caractérisés en ce que $A_1$ et $A_2$ sont des acides aminés naturels.

**7.** Dérivés selon la revendication 6, caractérisés en ce que $A_1$ est un résidu glycyle.

**8.** Dérivés selon la revendication 6, caractérisés en ce que $A_1$ est choisi parmi les résidus L-alanyle et L-valyle.

**9.** Dérivés selon la revendication 6, caractérisés en ce que $A_1$ est choisi parmi les résidus L-glycyle, L-alanyle et L-valyle, et $A_2$ parmi les résidus glycyle, L-phénylalanyle, L-histidyle, L-leucyle, L-valyle et L-alanyle.

**10.** Dérivés de L-proline, choisis parmi les cinnamoyl-glycyl-L-phénylalanyl-L-prolinamide, 4-fluorocinnamoyl-glycyl-L-phénylalanyl-L-prolinamide, 3,4-méthylènedioxy-cinnamoyl-glycyl-L-prolinamide, 3,4,5-triméthoxy-cinnamoylglycyl-L-phénylalanyl-L-prolinamide, cinnamoyl-glycyl-L-leucyl-L-prolinamide, benzoyl-glycyl-L-phénylalanyl-L-prolinamide-phénylacétyl-glycyl-L-phénylalanyl-L-prolinamide et cinnamoyl-glycyl-L-phénylalanyl-L-3,3' diméthyl-prolinamide.

**11.** Procédé de préparation de compositions pharmaceutiques, caractérisé en ce qu'on mélange une quantité efficace d'au moins un dérivé de L-proline de formule I, selon la revendication 1 ou leurs sels, avec un véhicule pharmaceutique.

**12.** Procédé selon la revendication 13, caractérisé en ce que le véhicule pharmaceutique est choisi pour une administration par voie orale, rectale, nasale, injectable.

**13.** Procédé selon la revendication 11, caractérisé en ce qu'on forme des tablettes, comprimés, gélules, gouttes, pilules, liposomes renfermant de 1 à 100 mg de principe actif par unité de prise, de préférence de 2,5 à 50 mg.

**14.** Procédé selon la revendication 13, caractérisé en ce qu'on forme des solutions injectables, ces solutions renfermant avantageusement, par unité de prise, de 1 à 50 mg de dérivé de L-proline, de préférence de 0,5 à 50 mg.

**15.** Réactif biologique, caractérisé en ce qu'il est élaboré à partir d'un dérivé de L-proline selon l'une quelconque des revendications 1 à 10.

**16.** Procédé de préparation de dérivés de L-proline selon la revendication 1, caractérisé par le fait qu'on fait réagir

- un dérivé de formule III :

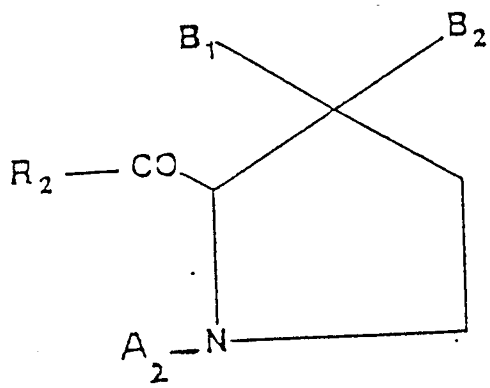

(III)

- avec un dérivé de formule IV

  $R_1$ - $A_1$ (IV)

  dans lesquelles $B_1$, $B_2$, $A_1$, $A_2$, $R_1$ et $R_2$ sont tels que définis dans la revendication 1.

**17.** Procédé selon la revendication 16, caractérisé en ce que pour obtenir le dérivé de formule III, on met en oeuvre un dérivé de proline de formule V :

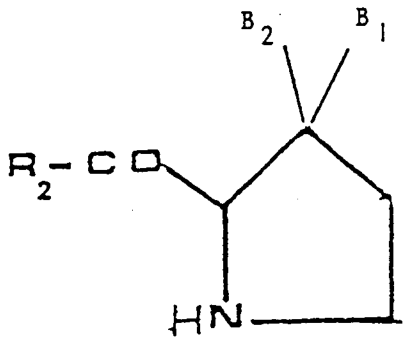

(V)

et un acide aminé $A_2$,
$B_1$, $B_2$, $R_2$ et $A_2$ étant tels que définis dans la revendication 1.

**18.** Procédé selon la revendication 16 ou 17, caractérisé en ce que le dérivé $R_1$-$A_1$, de formule IV est obtenu par condensation de dérivés actifs de $R_1$ et de $A_1$, la fonction carboxyle de $A_1$ étant bloquée par un groupe protecteur, ladite condensation étatn suivie d'une saponification à l'aide d'une base forte.

## Claims

**Claims for the following Contracting States : AT, BE, CH, DE, ES, FR, GB, IT, LI, LU, NL, SE**

**1.** Derivatives of L-proline, characterized in that they correspond to the Formula I :

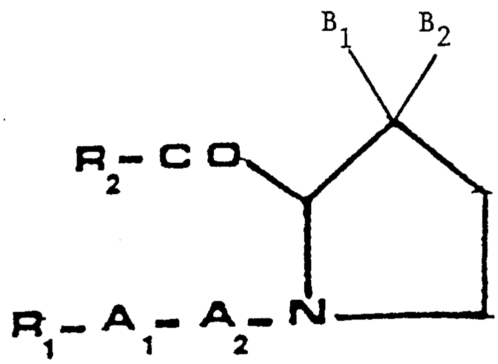

(I)

in which

- $R_1$ is a group corresponding to Formula II:

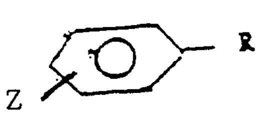

(II)

in which R is a carbonyl radical CO-, an acyl radical Y-CO-or an oxy-acyl radical O-Y-CO-, in which Y is an alkyl or alkenyl chain, containing in particular from 1 to 4 carbon atoms, Z

represents one or more hydrogen atoms, or one or more substituents in the ortho and/or ortho' and/or meta and/or meta' and/or para positions, chosen from among halogen atoms, $CF_3$ group, alkyl or alkoxy radicals containing from 1 to 4 carbon atoms and, in the case of 2 neighbouring susbstituents, an alkylenedioxy group, in which the alkylene group contains from 1 to 3 carbon atoms,

- $R_2$ is a $NH_2$ or OH radical or functional derivatives of these radicals,
- $A_1$ and $A_2$, identical or different from each other, are amino acid residues, and
- $B_1$ and $B_2$, identical or different from each other, represent a hydrogen atom or methyl group, and the pharmacologically acceptable salts of these derivatives, with the proviso that, on the one hand, $A_1$ be different from a phenylalanyl residue when $B_1$ and $B_2$ represent a hydrogen atom, $R_2$ represents an -OH group, $R_1$ represents a benzoyl radical and $A_2$ is an alanyl residue and, on the other hand, that $A_2$ be different from a proline residue, when R represents a -CO or -YCO group wherein Y is an alkyl group, $B_1$ and $B_2$ represent a hydrogen atom and $R_2$ is a -OH or -$NH_2$ group or the functional derivatives thereof.

**2.** Derivatives according to Claim 1, wherein Z in Formula II is a hydrogen atom.

**3.** Derivatives according to Claim 1, wherein Z in Formula II is a halogen atom selected from among chlorine and fluorine, a $CF_3$ group, an alkoxy radical chosen from amoung methoxy and ethoxy, and in the case of two neighbouring substituents, an alkylenedioxy radical chosen from among 3,4-methylene and 3,4-ethylenedioxy.

**4.** Derivatives according to any one of the Claims 1 to 3, wherein in Formula II, R is chosen from among the groups CO-, $CH_2$-CO-, $CH_2$-$CH_2$-CO-, $CH_2$-$CH_2$-$CH_2$-CO-, CH = CH-CO- or O-$CH_2$-CO-.

**5.** Derivatives according to claim 1, wherein $R_1$ is an optionally substituted cinnamoyl group.

**6.** Derivatives according to any one of the Claims 1 to 5, wherein $A_1$ and $A_2$ are naturally occurring amino acids.

**7.** Derivatives according to claim 6, wherein $A_1$ is a glycyl radical.

**8.** Derivatives according to claim 6, wherein $A_1$ is chosen from among L-alanyl and L-valyl residues.

**9.** Derivatives according to claim 6, wherein $A_1$ is chosen from among the residues L-glycyl, L-alanyl and L-valyl and $A_2$ from among the residues glycyl, L-phenylalanyl L-histidyl, L-leucyl, L-valyl and L-alanyl.

**10.** Derivatives of L-proline selected from the group comprising :
cinnamoyl-glycyl-L-phenylalanyl-L-prolinamide, 4-fluorocinnamoyl-glycyl-L-phenylalanyl-L-prolinamide, 3,4-methylenedioxycinnamoyl-glycyl-L-prolinamide, 3,4,5-trimethoxycinnamoyl-glycyl-L-phenylalanyl-L-prolinamide,cinnamoyl-glycyl-L-leucyl-L-prolinamide, benzoyl-glycycl-L-phenylalanyl-L-prolinamide, phenylacetyl glycyl-L-phenylalanyl-L-prolinamide and cinnamoyl-glycyl-L-phenylalanyl-L-3,3'-dimethyl-prolinamide.

**11.** Pharmaceutical composition, characterized in that it contains an efficacious amount of at least one L-proline derivative according to any one of the Claims 1 to 10, in combination with a pharmaceutical vehicle.

**12.** Composition according to Claim 11, characterized in that it can be administered by the oral, rectal or nasal route or by injection.

**13.** Composition according to Claim 11, characterized in that it relates to lozenges, tablets, gelatine capsules, drops, pills, liposomes, and in that it contains from 1 to 100 mg, and preferably from 2.5 to 50 mg, of the active principle per dosage unit.

**14.** Composition according to Claim 11, characterized in that it relates to injectable solutions, these solutions advantageously containing from 1 to 50 mg, and preferably from 0.5 to 50 mg of L-proline derivative per dosage unit.

**15.** Biological reagent, characterized in that it is based on a L-proline derivative according to any one of the Claims 1 to 10.

**16.** Process for the preparation of derivatives of L-proline according to Claim 1, wherein

- a derivative corresponding to Formula III :

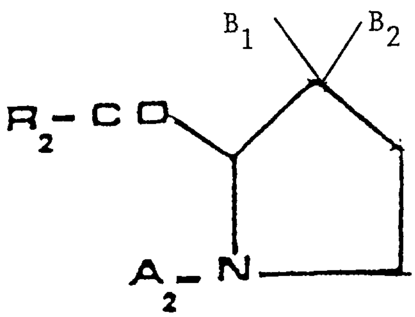

(III)

is allowed to react with a derivative corresponding to Formula IV :

$R_1$ - $A_1$ (IV)

in which $B_1$, $B_2$, $A_1$, $A_2$, $R_1$, and $R_2$ are such as defined in Claim I.

**17.** A process according to Claim 16, wherein, in order to obtain the derivative corresponding to Formula III, a proline derivative corresponding to Formula V :

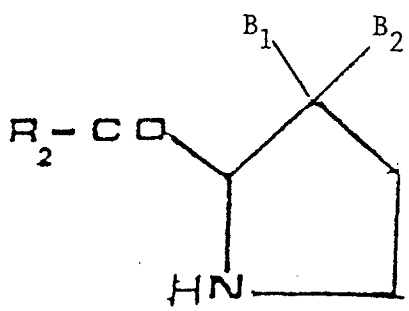

(V)

is condensed with an amino acid A2,
$B_1$, $B_2$, $R_2$ and $A_2$ being such as defined in Claim 1.

**18.** A process according to Claim 16 or 17 wherein the derivative $R_1$-$A_1$ of Formula IV is obtained by condensation of reactive derivatives of $R_1$ and of $A_1$, the carboxyl function of $A_1$ being blocked by a protecting group and the said condensation being followed by saponification using a strong base.

**Claims for the following Contracting State : ES**

**1.** Process for the preparation of derivatives of L-proline wherein

- a derivative corresponding to Formula III :

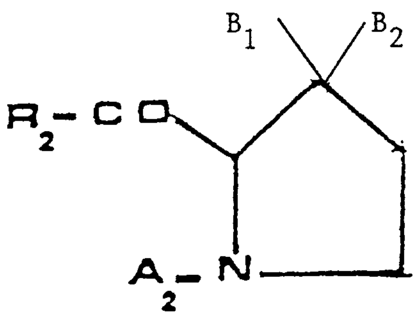

(III)

is allowed to react with a derivative corresponding to Formula IV :

$R_1$ - $A_1$ (IV)

which results in the obtention of proline derivatives of L-proline of Formula I :

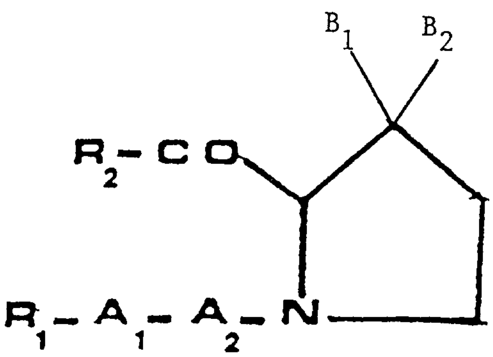

(I)

the substituents having in said formulae the following meanings :

- $R_1$ is a group corresponding to Formula II :

(II)

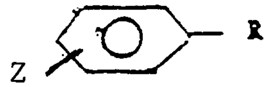

in which R is a carbonyl radical CO-, an acyl radical Y-CO-or an oxy-acyl radical O-Y-CO-, in which Y is an alkyl or alkenyl chain, containing in particular from 1 to 4 carbon atoms, Z represents one or more hydrogen atoms, or one or more substituents in the ortho and/or ortho' and/or meta and/or meta' and/or para positions, chosen from among halogen atoms, $CF_3$ group, alkyl or alkoxy radicals containing from 1 to 4 carbon atoms and in the case of 2 neighbouring susbstituents, an alkylenedioxy group, in which the alkylene group contains from 1 to 3 carbon atoms,

- $R_2$ is a $NH_2$ or OH radical or functional derivatives of these radicals,
- $A_1$ and $A_2$, identical or different, are amino acid residues, and
- $B_1$ and $B_2$, identical or different, represent a hydrogen atom or methyl group, and the pharmacologically acceptable salts of these derivatives, with the proviso that, on the one hand, A1 be different from a phenylalanyl residue when $B_1$ and $B_2$ represent an hydrogen atom, $R_2$ represents

an -OH group, $R_1$ represents a benzoyl radical and $A_2$ is an alanyl residue and, on the other hand, that $A_2$ be different from a proline residue, when R represents a -CO or -YCO group wherein Y is alkyl group, $B_1$ and $B_2$ represent an hydrogen atom and $R_2$ is a -OH or $-NH_2$ group or the functional derivatives thereof.

**2.** A process according to Claim 1, wherein Z, in Formula II, is a hydrogen atom.

**3.** A process according to Claim 1, wherein Z in Formula II is a halogen atom selected from among chlorine and fluorine, a $CF_3$ group, an alkoxy radical chosen from amoung methoxy and ethoxy, and in the case of two neighbouring substituents, an alkylenedioxy radical chosen from among 3,4-methylene and 3,4-ethylenedioxy.

**4.** A process according to anyone of the Claims 1 to 3, wherein in Formula II, R is chosen from among the groups CO-, $CH_2$-CO-, $CH_2$-$CH_2$-CO-, $CH_2$-$CH_2$-$CH_2$-CO-, CH=CH-CO- or O-$CH_2$-CO.

**5.** A process according to claim 1, wherein $R_1$ is an optionally substituted cinnamoyl group.

**6.** A process according to anyone of the Claims 1 to 5, wherein $A_1$ and $A_2$ are naturally occurring amino acids.

**7.** A process according to claim 6, wherein $A_1$ is a glycyl radical.

**8.** A process according to claim 6, wherein $A_1$ is chosen from among L-alanyl and L-valyl residues.

**9.** A process according to claim 6, wherein $A_1$ is chosen from among the residues L-glycyl, L-alanyl and L-valyl and $A_2$ from among the residues L-glycyl, L-phenylalanyl, L-histidyl, L-leucyl, L-valyl and L-alanyl.

**10.** A process according to claim 1, wherein the products of formula I are obtained which are selected from cinnamoyl-glycyl-L-phenylalanyl-L-prolinamide, 4-fluorocinnamoyl-glycyl-L-phenylalanyl-L-prolinamide, 3,4-methylenedioxycinnamoyl-glycyl-L-prolinamide, 3,4,5-trimethoxycinnamoyl-glycyl-L-phenylalanyl-L-prolinamide,cinnamoyl-glycyl-L-leucyl-L-prolinamide, benzoyl-glycyl-L-phenylalanyl-L-prolinamide, phenylacetyl-glycyl-L-phenylalanyl-L-prolinamide and cinnamoyl-glycyl-L-phenylalanyl-L-3,3'-dimethyl-prolinamide.

**11.** A process according to anyone of the Claims 1 to 10, wherein, in order to obtain the derivative corresponding to Formula III, a proline derivative corresponding to Formula V :

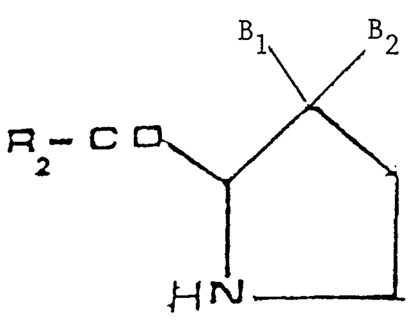

(V)

is condensed with an amino acid A2,
$B_1$, $B_2$, $R_2$ and $A_2$ being such as defined in Claim 1.

**12.** A process according to anyone of the preceding Claims, wherein the derivative $R_1$-$A_1$ of Formula IV is obtained by condensation of reactive derivatives of $R_1$ and of $A_1$, the carboxyl function of $A_1$ being blocked by a protecting group and the said condensation being followed by saponification using a strong base.

**13.** Process for the preparation of pharmaceutical compositions, wherein an efficacious amount of at least one L-proline derivative of formula I, according to claim 1, or the salts are mixed with a pharmaceutical vehicle.

**14.** A process according to Claim 13, wherein the pharmaceutical vehicle is chosen for an administration by the oral, rectal or nasal route or by injection.

**15.** A process according to Claim 13, wherein lozenges, tablets, gelatine capsules, drops, pills, liposomes, are prepared and contain from 1 to 100 mg, and preferably from 2.5 to 50 mg, of the active principle per dosage unit.

**16.** A process according to Claim 13, wherein injectable solutions are prepared, these solutions advantageously containing from 0,5 to 50 mg, and preferably from 1 to 50 mg of L-proline derivative per dosage unit.

**17.** Use of L-proline derivatives obtained according to any one of the Claims 1 to 12 as biological reagent.

**Claims for the following Contracting State : GR**

**1.** Derivatives of L-proline, characterized in that they correspond to the Formula I :

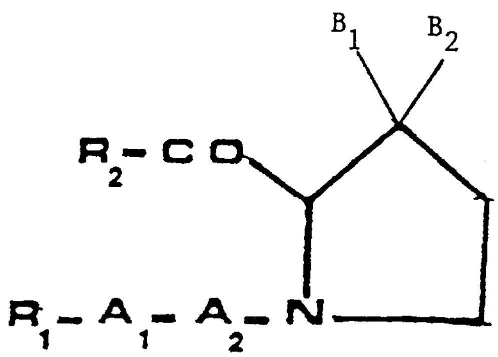

(I)

in which
- $R_1$ is a group corresponding to Formula II :

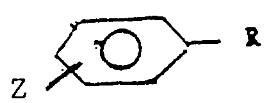

(II)

in which R is a carbonyl radical CO-, an acyl radical Y-CO-or an oxy-acyl radical O-Y-CO-, in which Y is an alkyl or alkenyl chain, containing in particular from 1 to 4 carbon atoms, Z represents one or more hydrogen atoms, or one or more substituents in the ortho and/or ortho' and/or meta and/or meta' and/or para positions, chosen from among halogen atoms, $CF_3$ group, alkyl or alkoxy radicals containing from 1 to 4 carbon atoms and, in the case of 2 neighbouring susbstituents, an alkylenedioxy group, in which the alkylene group contains from 1 to 3 carbon atoms,
- $R_2$ is a $-NH_2$ or -OH radical or functional derivatives of these radicals,
- $A_1$ and $A_2$, identical or different from each other, are amino acid residues, and
- $B_1$ and $B_2$, identical or different from each other, represent a hydrogen atom or methyl group, and the pharmacologically acceptable salts of these derivatives, with the proviso that, on the one hand, A1 be different from a phenylalanyl residue when $B_1$ and $B_2$ represent a hydrogen atom, $R_2$ represents an -OH group, $R_1$ represents a benzoyl radical and $A_2$ is an alanyl residue and, on the other hand, that $A_2$ be different from a proline residue, when R represents a -CO or -YCO

group wherein Y is an alkyl group, $B_1$ and $B_2$ represent a hydrogen atom and $R_2$ is a -OH or $-NH_2$ group or the functional derivatives thereof.

**2.** Derivatives according to Claim 1, wherein Z in Formula II is a hydrogen atom.

**3.** Derivatives according to Claim 1, wherein Z in Formula II is a halogen atom selected from among chlorine and fluorine, a $CF_3$ group, an alkoxy radical chosen from amoung methoxy and ethoxy, and in the case of two neighbouring substituents, an alkylenedioxy radical chosen from among 3,4-methylene and 3,4-ethylenedioxy.

**4.** Derivatives according to anyone of the Claims 1 to 3, wherein in Formula II, R is chosen from among the groups CO-, $CH_2$-CO-, $CH_2$-$CH_2$-CO-, $CH_2$-$CH_2$-$CH_2$-CO-, CH = CH-CO- or O-$CH_2$-CO-.

**5.** Derivatives according to claim 1, wherein $R_1$ is an optionally substituted cinnamoyl group.

**6.** Derivatives according to anyone of the Claims 1 to 5, wherein $A_1$ and $A_2$ are naturally occurring amino acids.

**7.** Derivatives according to claim 6, wherein $A_1$ is a glycyl radical.

**8.** Derivatives according to claim 6, wherein $A_1$ is chosen from among L-alanyl and L-valyl residues.

**9.** Derivatives according to claim 6, wherein $A_1$ is chosen from among the residues L-glycyl, L-alanyl and L-valyl and $A_2$ from among the residues glycyl, L-phenylalanyl L-histidyl, L-leucyl, L-valyl and L-alanyl.

**10.** Derivatives of L-proline selected from the group comprising :
cinnamoyl-glycyl-L-phenylalanyl-L-prolinamide, 4-fluorocinnamoyl-glycyl-L-phenylalanyl-L-prolinamide, 3,4-methylenedioxycinnamoyl-glycyl-L-prolinamide, 3,4,5-trimethoxycinnamoyl-glycyl-L-phenylalanyl-L-prolinamide,cinnamoyl-glycyl-L-leucyl-L-prolinamide, benzoyl-glycyl-L-phenylalanyl-L-prolinamide, phenylacetylglycyl-L-phenylalanyl-L-prolinamide and cinnamoyl-glycyl-L-phenylalanyl-L-3,3'-dimethyl-prolinamide.

**11.** A process for the preparation of pharmaceutical compositions, wherein an efficacious amount of at least one of L-proline derivative of formula I according to Claim 1 or the salts thereof is mixed with a pharmaceutical vehicle.

**12.** A process according to Claim 13, wherein the pharmaceutical vehicle is chosen for an administration by the oral, rectal or nasal route or by injection.

**13.** A process according to Claim 11, wherein lozenges, tablets, gelatine capsules, drops, pills, liposomes, are prepared and contain from 1 to 100 mg, and preferably from 2.5 to 50 mg, of the active principle per dosage unit.

**14.** A process according to Claim 11, wherein injectable solutions, are prepared, these solutions advantageously containing from 0,5 to 50 mg, and preferably from 1 to 50 mg of L-proline derivative per dosage unit.

**15.** Biological reagent, characterized in that it is based on a L-proline derivative according to any one of the Claims 1 to 10.

**16.** Process for the preparation of derivatives of L-proline according to Claim 1, wherein

- a derivative corresponding to Formula III :

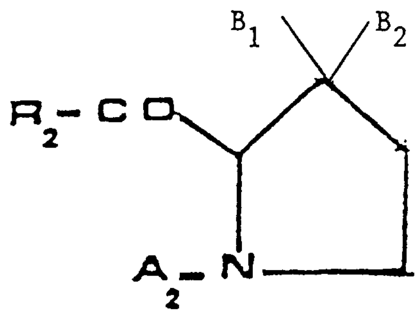

(III)

is allowed to react with a derivative corresponding to Formula IV :

$R_1$ - $A_1$ (IV)

in which $B_1$, $B_2$, $A_1$, $A_2$, $R_1$, and $R_2$ are such as defined in Claim I.

**17.** A process according to Claim 16, wherein, in order to obtain the derivative corresponding to Formula III, a proline derivative corresponding to Formula V :

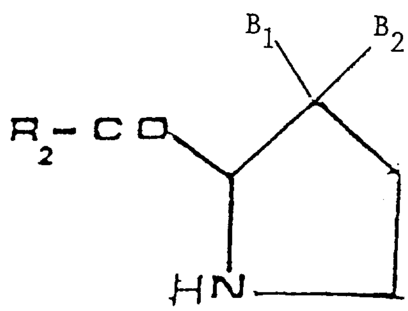

(V)

is condensed with an amino acid A2,
$B_1$, $B_2$, $R_2$ and $A_2$ being such as defined in Claim 1.

**18.** A process according to Claim 16 or 17 wherein the derivative $R_1$-$A_1$ of Formula IV is obtained by condensation of reactive derivatives of $R_1$ and of $A_1$, the carboxyl function of $A_1$ being blocked by a protecting group and the said condensation being followed by saponification using a strong base.

**Patentansprüche**
**Patensprüche für folgende Vertragsstaaten : AT, BE, CH, DE, ES, FR, GB, IT, LI, LU, NL, SE**

**1.** L-Prolin-Derivate, dadurch gekennzeichnet, daß sie der Formel I:

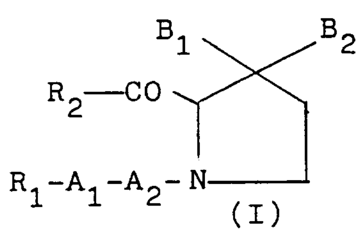

(I)

entsprechen, worin

- $R_1$ eine Gruppe der Formel II

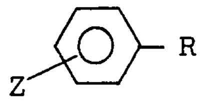

(II)

darstellt, worin R einen Carbonylrest CO-, einen Acylrest Y-CO- oder einen Oxyacylrest O-Y-CO- bedeutet, worin Y eine Alkyl- oder Alkenylkette mit insbesondere 1 bis 4 Kohlenstoffatomen bedeutet, Z ein oder mehrere Wasserstoffatome oder einen oder mehrere Substituenten in den Stellungen ortho und/oder ortho' und/oder meta und/oder meta' und/oder para, ausgewählt unter Halogenatomen, einer $CF_3$-Gruppe, den Alkyl- oder Alkoxyresten mit 1 bis 4 Kohlenstoffatomen und, bei zwei benachbarten Substituenten, einer Alkylendioxygruppe, darstellt, wobei die Alkylengruppe 1 bis 3 Kohlenstoffatome umfaßt,
- $R_2$ einen Rest $NH_2$ oder OH darstellt, oder funktionelle Derivate dieser Reste,
- $A_1$ und $A_2$, die gleich oder voneinander verschieden sind, Aminosäurereste darstellen und
- $B_1$ und $B_2$, die gleich oder voneinander verschieden sind, ein Wasserstoffatom oder eine Methylgruppe bedeuten,

und die pharmakologisch annehmbaren Salze dieser Derivate, mit der Maßgabe, daß einerseits $A_1$ von einem Phenylalanylrest verschieden ist, wenn $B_1$ und $B_2$ ein Wasserstoffatom bedeuten, $R_2$ eine Gruppe -OH darstellt, $R_1$ einen Benzoylrest bedeutet und $A_2$ einen Alanylrest bezeichnet, und anderseits $A_2$ von einem Prolinrest verschieden ist, wenn R eine Gruppe -CO oder -YCO darstellt, worin Y eine Alkylgruppe bedeutet, $B_1$ und $B_2$ ein Wasserstoffatom bezeichnen und $R_2$ eine -OH-Gruppe oder $-NH_2$-Gruppe oder ihre funktionellen Derivate bezeichnet.

**2.** Derivate nach Anspruch 1, dadurch gekennzeichnet, daß Z in der Formel II ein Wasserstoffatom bedeutet.

**3.** Derivate nach Anspruch 1, dadurch gekennzeichnet, daß Z in der Formel II ein unter Chlor oder Fluor ausgewähltes Halogenatom, eine $CF_3$-Gruppe, einen unter Methoxy oder Ethoxy ausgewählten Alkoxyrest und, für zwei benachbarte Positionen, einen unter 3,4-Methylendioxy oder 3,4-Ethylendioxy ausgewählten Alkylendioxyrest bedeutet.

**4.** Derivate nach einem der Ansprüche 1 bis 3, dadurch gekennzeichnet, daß R in Formel II unter den Gruppen CO-, $CH_2$-CO-, $CH_2$-$CH_2$-CO-, $CH_2$-$CH_2$-$CH_2$-CO-, CH = CH-CO- oder O-$CH_2$-CO- ausgewählt ist.

**5.** Derivate nach Anspruch 1, dadurch gekennzeichnet, daß $R_1$ eine gegebenenfalls substituierte Cinnamoylgruppe bedeutet.

**6.** Derivate nach einem der Ansprüche 1 bis 5, dadurch gekennzeichnet, daß $A_1$ und $A_2$ natürliche Aminosäuren sind.

**7.** Derivate nach Anspruch 6, dadurch gekennzeichnet, daß $A_1$ einen Glycylrest bedeutet.

**8.** Derivate nach Anspruch 6, dadurch gekennzeichnet, daß $A_1$ unter den L-Alanyl- und L-Valylresten ausgewählt ist.

**9.** Derivate nach Anspruch 6, dadurch gekennzeichnet, daß $A_1$ unter den Resten L-Glycyl, L-Alanyl und L-Valyl ausgewählt ist und $A_2$ unter den Resten Glycyl, L-Phenylalanyl, L-Histidyl, L-Leucyl, L-Valyl und L-Alanyl ausgewählt ist.

**10.** L-Prolin-Derivate, ausgewählt unter Cinnamoyl-glycyl-L-phenylalanyl-L-prolinamid, 4-Fluorcinnamoyl-glycyl-L-phenylalanyl-L-prolinamid, 3,4-Methylendioxycinnamoyl-glycyl-L-prolinamid, 3,4,5-Trimethoxycinnamoyl-glycyl-L-phenylalanyl-L-prolinamid, Cinnamoyl-glycyl-L-leucyl-L-prolinamid,

Benzoyl-glycyl-L-phenylalanyl-L-prolinamid, Phenylacetyl-glycyl-L-phenylalanyl-L-prolinamid und Cinnamoyl-glycyl-L-phenylalanyl-L-3,3'-dimethyl-prolinamid.

**11.** Pharmazeutische Zusammensetzung, dadurch gekennzeichnet, daß sie eine wirksame Menge wenigstens eines L-Prolinderivats nach einem der Ansprüche 1 bis 10 im Gemisch mit einem pharmazeutischen Träger enthält.

**12.** Zusammensetzung nach Anspruch 11, dadurch gekennzeichnet, daß sie auf oralem, rektalem oder nasalem Weg verabreichbar oder injizierbar ist.

**13.** Zusammensetzung nach Anspruch 11, dadurch gekennzeichnet, daß es sich um Tabletten, Kapseln, Tropfen, Pillen oder Liposome handelt und daß sie 1 bis 100 mg Wirkstoff je Dosiseinheit, vorzugsweise 2,5 bis 50 mg enthält.

**14.** Zusammensetzung nach Anspruch 11, dadurch gekennzeichnet, daß es sich um injizierbare Lösungen handelt, welche Lösungen vorteilhaft je Dosiseinheit 1 bis 50 mg L-Prolinderivat, vorzugsweise 0,5 bis 50 mg enthalten.

**15.** Biologisches Reaktionsmittel, dadurch gekennzeichnet, daß es, ausgehend von einem L-Prolinderivat nach einem der Ansprüche 1 bis 10, erarbeitet ist.

**16.** Verfahren zur Herstellung von L-Prolinderivaten nach Anspruch 1, dadurch gekennzeichnet, daß man ein Derivat der Formel III:

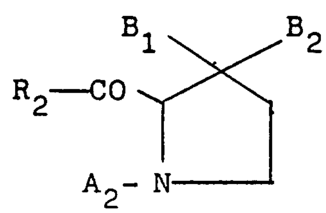

(III)

mit einem Derivat der Formel IV

$R_1$ - $A_1$ (IV),

worin $B_1$, $B_2$, $A_1$, $A_2$, $R_1$ und $R_2$ wie in Anspruch 1 definiert sind, miteinander reagieren läßt.

**17.** Verfahren nach Anspruch 16, dadurch gekennzeichnet, daß man zur Gewinnung des Derivats der Formel III ein Prolinderivat der Formel V:

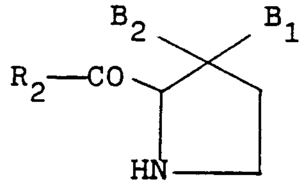

(V)

und eine Aminosäure $A_2$ einsetzt, wobei $B_1$, $B_2$, $R_2$ und $A_2$ wie in Anspruch 1 definiert sind.

**18.** Verfahren nach Anspruch 16 oder 17, dadurch gekennzeichnet, daß das Derivat $R_1$-$A_1$ der Formel IV durch Kondensation von reaktionsfähigen Derivaten von $R_1$ und von $A_1$ erhalten wird, wobei die Carboxylfunktion von $A_1$ durch eine Schutzgruppe blockiert ist, nach welcher Kondensation eine

Verseifung mit Hilfe einer starken Base vorgenommen wird.

**Patensprüche für folgenden Vertragsstaat : ES**

**1.** Verfahren zur Herstellung von L-Prolin-Derivaten, dadurch gekennzeichnet, daß man ein Derivat der Formel III:

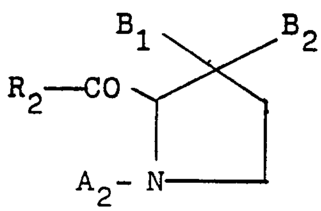

(III)

mit einem Derivat der Formel IV

$R_1$ - $A_1$ (IV)

zu Prolinderivaten der Formel I:

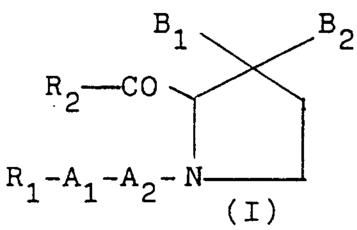

(I)

umsetzt, wobei die Substituenten in diesen Formeln die folgenden Bedeutungen aufweisen:

- $R_1$ bedeutet eine Gruppe der Formel II

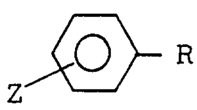

(II) ,

worin R einen Carbonylrest CO-, einen Acylrest Y-CO- oder einen Oxyacylrest O-Y-CO- bedeutet, worin Y eine Alkyl- oder Alkenylkette mit insbesondere 1 bis 4 Kohlenstoffatomen bedeutet, Z ein oder mehrere Wasserstoffatome oder einen oder mehrere Substituenten in den Stellungen ortho und/oder ortho' und/oder meta und/oder meta' und/oder para, ausgewählt unter Halogenatomen, einer $CF_3$-Gruppe, den Alkyl- oder Alkoxyresten mit 1 bis 4 Kohlenstoffatomen und, bei zwei benachbarten Substituenten, einer Alkylendioxygruppe, darstellt, wobei die Alkylengruppe 1 bis 3 Kohlenstoffatome umfaßt,
- $R_2$ stellt einen Rest $NH_2$ oder OH, oder funktionelle Derivate dieser Reste dar,
- $A_1$ und $A_2$, die gleich oder voneinander verschieden sind, stellen Aminosäurereste dar und
- $B_1$ und $B_2$, die gleich oder voneinander verschieden sind, bedeuten ein Wasserstoffatom oder eine Methylgruppe,

und die pharmakologisch annehmbaren Salze dieser Derivate, mit der Maßgabe, daß einerseits $A_1$ von einem phenylalanylrest verschieden ist, wenn $B_1$ und $B_2$ ein Wasserstoffatom bedeuten, $R_2$ eine Gruppe -OH darstellt, $R_1$ einen Benzoylrest bedeutet und $A_2$ einen Alanylrest bezeichnet, und anderseits $A_2$ von einem Prolinrest verschieden ist, wenn R eine Gruppe -CO oder -YCO darstellt, worin Y eine Alkylgruppe bedeutet, $B_1$ und $B_2$ ein Wasserstoffatom bezeichnen und $R_2$ eine -OH-Gruppe

oder $-NH_2$-Gruppe oder ihre funktionellen Derivate bezeichnet.

**2.** Verfahren nach Anspruch 1, dadurch gekennzeichnet, daß Z in der Formel II ein Wasserstoffatom bedeutet.

**3.** Verfahren nach Anspruch 1, dadurch gekennzeichnet, daß Z in der Formel II ein unter Chlor oder Fluor ausgewähltes Halogenatom, eine $CF_3$-Gruppe, einen unter Methoxy oder Ethoxy ausgewählten Alkoxyrest und, für zwei benachbarte Positionen, einen unter 3,4-Methylendioxy oder 3,4-Ethylendioxy ausgewählten Alkylendioxyrest bedeutet.

**4.** Verfahren nach einem der Ansprüche 1 bis 3, dadurch gekennzeichnet, daß R in Formel II unter den Gruppen CO-, $CH_2$-CO-, $CH_2$-$CH_2$-CO-, $CH_2$-$CH_2$-$CH_2$-CO-, CH = CH-CO- oder O-$CH_2$-CO- ausgewählt ist.

**5.** Verfahren nach Anspruch 1, dadurch gekennzeichnet, daß $R_1$ eine gegebenenfalls substituierte Cinnamoylgruppe bedeutet.

**6.** Verfahren nach einem der Ansprüche 1 bis 5, dadurch gekennzeichnet, daß $A_1$ und $A_2$ natürliche Aminosäuren sind.

**7.** Verfahren nach Anspruch 6, dadurch gekennzeichnet, daß $A_1$ einen Glycylrest bedeutet.

**8.** Verfahren nach Anspruch 6, dadurch gekennzeichnet, daß $A_1$ unter den L-Alanyl- und L-Valylresten ausgewählt ist.

**9.** Verfahren nach Anspruch 6, dadurch gekennzeichnet, daß $A_1$ unter den Resten L-Glycyl, L-Alanyl und L-Valyl ausgewählt ist und $A_2$ unter den Resten Glycyl, L-Phenylalanyl, L-Histidyl, L-Leucyl, L-Valyl und L-Alanyl ausgewählt ist.

**10.** Verfahren nach Anspruch 1, dadurch gekennzeichnet, daß man Produkte der Formel I erhält, ausgewählt unter Cinnamoyl-glycyl-L-phenylalanyl-L-prolinamid, 4-Fluorcinnamoyl-glycyl-L-phenylalanyl-L-prolinamid, 3,4-Methylendioxycinnamoyl-glycyl-L-prolinamid, 3,4,5-Trimethoxycinnamoyl-glycyl-L-phenylalanyl-L-prolinamid, Cinnamoyl-glycyl-L-leucyl-L-prolinamid, Benzoyl-glycyl-L-phenylalanyl-L-prolinamide, Phenylacetyl-glycyl-L-phenylalanyl-L-prolinamid und Cinnamoyl-glycyl-L-phenylalanyl-L-3,3'-dimethyl-prolinamid.

**11.** Verfahren nach einem der Ansprüche 1 bis 10, dadurch gekennzeichnet, daß man zur Gewinnung des Derivats der Formel III ein Prolinderivat der Formel V:

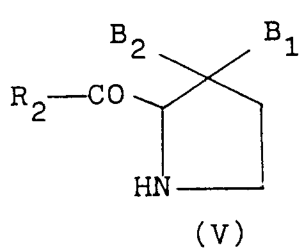

(V)

und eine Aminosäure $A_2$ einsetzt, wobei $B_1$, $B_2$, $R_2$ und $A_2$ wie in Anspruch 1 definiert sind.

**12.** Verfahren nach einem der vorstehenden Ansprüche, dadurch gekennzeichnet, daß das Derivat $R_1$-$A_1$ der Formel IV durch Kondensation von reaktionsfähigen Derivaten von $R_1$ und von $A_1$ erhalten wird, wobei die Carboxylfunktion von $A_1$ durch eine Schutzgruppe blockiert ist, nach welcher Kondensation eine Verseifung mit Hilfe einer starken Base vorgenommen wird.

**13.** Verfahren zur Herstellung von pharmazeutischen Zusammensetzungen, dadurch gekennzeichnet, daß man eine wirksame Menge wenigstens eines L-Prolinderivats der Formel I nach Anspruch 1 oder seine

Salze mit einem pharmazeutischen Träger vermischt.

**14.** Verfahren nach Anspruch 13, dadurch gekennzeichnet, daß der pharmazeutische Träger für eine Verabreichung auf oralem, rektalem oder nasalem Weg oder durch Injektion gewählt wird.

**15.** Verfahren nach Anspruch 13, dadurch gekennzeichnet, daß man Tabletten, Kapseln, Tropfen, Pillen, Liposome mit einem Gehalt an 1 bis 100 mg Wirkstoff je Dosiseinheit, vorzugsweise 2,5 bis 50 mg ausbildet.

**16.** Verfahren nach Anspruch 13, dadurch gekennzeichnet, daß man injizierbare Lösungen ausbildet, die vorzugsweise je Dosiseinheit 1 bis 50 mg L-Prolinderivat, vorzugsweise 0,5 bis 50 mg enthalten.

**17.** Verwendung der nach einem der Ansprüche 1 bis 12 erhaltenen L-Prolinderivate als biologisches Reagens.

**Patensprüche für folgenden Vertragsstaat : GR**

**1.** L-Prolin-Derivate, dadurch gekennzeichnet, daß sie der Formel I:

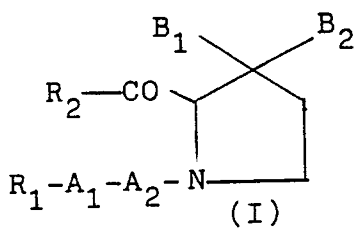

(I)

entsprechen, worin

- $R_1$ eine Gruppe der Formel II

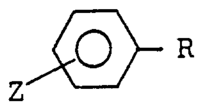

(II)

darstellt, worin R einen Carbonylrest CO-, einen Acylrest Y-CO- oder einen Oxyacylrest O-Y-CO- bedeutet, worin Y eine Alkyl- oder Alkenylkette mit insbesondere 1 bis 4 Kohlenstoffatomen bedeutet, Z ein oder mehrere Wasserstoffatome oder einen oder mehrere Substituenten in den Stellungen ortho und/oder ortho' und/oder meta und/oder meta' und/oder para, ausgewählt unter Halogenatomen, einer $CF_3$-Gruppe, den Alkyl- oder Alkoxyresten mit 1 bis 4 Kohlenstoffatomen und, bei zwei benachbarten Substituenten, einer Alkylendioxygruppe, darstellt, wobei die Alkylengruppe 1 bis 3 Kohlenstoffatome umfaßt,
- $R_2$ einen Rest $NH_2$ oder OH darstellt, oder funktionelle Derivate dieser Reste,
- $A_1$ und $A_2$, die gleich oder voneinander verschieden sind, Aminosäurereste darstellen und
- $B_1$ und $B_2$, die gleich oder voneinander verschieden sind, ein Wasserstoffatom oder eine Methylgruppe bedeuten,

und die pharmakologisch annehmbaren Salze dieser Derivate, mit der Maßgabe, daß einerseits $A_1$ von einem Phenylalanylrest verschieden ist, wenn $B_1$ und $B_2$ ein Wasserstoffatom bedeuten, $R_2$ eine Gruppe -OH darstellt, $R_1$ einen Benzoylrest bedeutet und $A_2$ einen Alanylrest bezeichnet, und anderseits $A_2$ von einem Prolinrest verschieden ist, wenn R eine Gruppe -CO oder -YCO darstellt, worin Y eine Alkylgruppe bedeutet, $B_1$ und $B_2$ ein Wasserstoffatom bezeichnen und $R_2$ eine -OH-Gruppe oder -$NH_2$-Gruppe oder ihre funktionellen Derivate bezeichnet.

2. Derivate nach Anspruch 1, dadurch gekennzeichnet, daß Z in der Formel II ein Wasserstoffatom bedeutet.

3. Derivate nach Anspruch 1, dadurch gekennzeichnet, daß Z in der Formel II ein unter Chlor oder Fluor ausgewähltes Halogenatom, eine $CF_3$-Gruppe, einen unter Methoxy oder Ethoxy ausgewählten Alkoxyrest und, für zwei benachbarte Positionen, einen unter 3,4-Methylendioxy oder 3,4-Ethylendioxy ausgewählten Alkylendioxyrest bedeutet.

4. Derivate nach einem der Ansprüche 1 bis 3, dadurch gekennzeichnet, daß R in Formel II unter den Gruppen CO-, $CH_2$-CO-, $CH_2$-$CH_2$-CO-, $CH_2$-$CH_2$-$CH_2$-CO-, CH = CH-CO- oder O-$CH_2$-CO- ausgewählt ist.

5. Derivate nach Anspruch 1, dadurch gekennzeichnet, daß $R_1$ eine gegebenenfalls substituierte Cinnamoylgruppe bedeutet.

6. Derivate nach einem der Ansprüche 1 bis 5, dadurch gekennzeichnet, daß $A_1$ und $A_2$ natürliche Aminosäuren sind.

7. Derivate nach Anspruch 6, dadurch gekennzeichnet, daß $A_1$ einen Glycylrest bedeutet.

8. Derivate nach Anspruch 6, dadurch gekennzeichnet, daß $A_1$ unter den L-Alanyl- und L-Valylresten ausgewählt ist.

9. Derivate nach Anspruch 6, dadurch gekennzeichnet, daß $A_1$ unter den Resten L-Glycyl, L-Alanyl und L-Valyl ausgewählt ist und $A_2$ unter den Resten Glycyl, L-Phenylalanyl, L-Histidyl, L-Leucyl, L-Valyl und L-Alanyl ausgewählt ist.

10. L-Prolin-Derivate, ausgewählt unter Cinnamoyl-glycyl-L-phenylalanyl-L-prolinamid, 4-Fluorcinnamoyl-glycyl-L-phenylalanyl-L-prolinamid, 3,4-Methylendioxycinnamoyl-glycyl-L-prolinamid. 3,4,5-Trimethoxycinnamoyl-glycyl-L-phenylalanyl-L-Prolinamid, Cinnamoyl-glycyl-L-leucyl-L-prolinamid, Benzoyl-glycyl-L-phenylalanyl-L-prolinamid, Phenylacetyl-glycyl-L-phenylalanyl-L-prolinamid und Cinnamoyl-glycyl-L-phenylalanyl-L-3,3'-dimethyl-prolinamid.

11. Verfahren zur Herstellung von pharmazeutischen Zusammensetzungen, dadurch gekennzeichnet, daß man eine wirksame Menge wenigstens eines L-Prolinderivats der Formel I nach Anspruch 1 oder seine Salze mit einem pharmazeutischen Träger vermischt.

12. Verfahren nach Anspruch 11, dadurch gekennzeichnet, daß der pharmazeutische Träger für eine Verabreichung auf oralem, rektalem oder nasalem Weg oder durch Injektion gewählt wird.

13. Verfahren nach Anspruch 11, dadurch gekennzeichnet, daß man Tabletten, Kapseln, Tropfen, Pillen, Liposome mit einem Gehalt an 1 bis 100 mg Wirkstoff je Dosiseinheit, vorzugsweise 2,5 bis 50 mg ausbildet.

14. Verfahren nach Anspruch 11, dadurch gekennzeichnet, daß man injizierbare Lösungen ausbildet, die vorteilhaft je Dosiseinheit 1 bis 50 mg L-Prolinderivat, vorzugsweise 0,5 bis 50 mg enthalten.

15. Biologisches Reaktionsmittel, dadurch gekennzeichnet, daß es, ausgehend von einem L-Prolinderivat nach einem der Ansprüche 1 bis 10, erarbeitet ist.

16. Verfahren zur Herstellung von L-Prolinderivaten nach Anspruch 1, dadurch gekennzeichnet, daß man ein Derivat der Formel III:

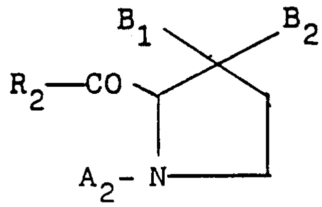

(III)

mit einem Derivat der Formel IV

$R_1 - A_1$ (IV),

worin $B_1$, $B_2$, $A_1$, $A_2$, $R_1$ und $R_2$ wie in Anspruch 1 definiert sind, miteinander reagieren läßt.

**17.** Verfahren nach Anspruch 16, dadurch gekennzeichnet, daß man zur Gewinnung des Derivats der Formel III ein Prolinderivat der Formel V:

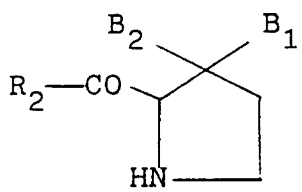

(V)

und eine Aminosäure $A_2$ einsetzt, wobei $B_1$, $B_2$, $R_2$ und $A_2$ wie in Anspruch 1 definiert sind.

**18.** Verfahren nach Anspruch 16 oder 17, dadurch gekennzeichnet, daß das Derivat $R_1$-$A_1$ der Formel IV durch Kondensation von reaktionsfähigen Derivaten von $R_1$ und von $A_1$ erhalten wird, wobei die Carboxylfunktion von $A_1$ durch eine Schutzgruppe blockiert ist, nach welcher Kondensation eine Verseifung mit Hilfe einer starken Base vorgenommen wird.